# EUROPÄISCHE PATENTSCHRIFT

(11) **EP 4 364 559 B1**
(45) Veröffentlichungstag und Bekanntmachung des Hinweises auf die Patenterteilung: **30.04.2025**
(21) Anmeldenummer: 23195751.5
(22) Anmeldetag: 06.09.2023
(51) Int. Cl.: A01K 67/36

(54) **VORRICHTUNG ZUR AUFZUCHT VON INSEKTENLARVEN UND VERFAHREN ZUR AUFZUCHT VON INSEKTENLARVEN**
DEVICE FOR REARING INSECT LARVAE AND METHOD FOR REARING INSECT LARVAE
DISPOSITIF D'ÉLEVAGE DE LARVES D'INSECTES ET PROCÉDÉ D'ÉLEVAGE DE LARVES D'INSECTES

(30) Priorität: 02.11.2022 DE 102022128970
(43) Veröffentlichungstag der Anmeldung: 08.05.2024
(73) Patentinhaber: Hermetia Tech UG, 52072 Aachen (DE)
(72) Erfinder: WENNING, Marius, 44577 Castrop-Rauxel (DE); SANDJON, Jérôme, 52353 Düren (DE)
(74) Vertreter: Richly & Ritschel Patentanwälte PartG mbB

(56) Entgegenhaltungen:
- EP-A1- 3 868 201
- CN-A- 106 962 292
- CN-A- 114 503 958
- CN-U- 210 900 996
- KR-B1- 102 078 152
- US-A1- 2020 253 176

## Beschreibung

Die Erfindung betrifft eine Vorrichtung zur Aufzucht und Ernte von Insektenlarven und ein Verfahren zur Aufzucht und Ernte von Insektenlarven.

Insektenlarven stellen eine nachhaltige Proteinquelle dar, die die Flächenkonkurrenz bestehender Agrarsysteme reduzieren kann. Mithilfe von Insektenlarven lassen sich Nahrungsmittelreste recyceln. Die Insektenlarven lassen sich als Futter- oder Nahrungsmittel einsetzen.

Die Nahrungsmittelindustrie produziert etwa ein Viertel der insgesamt anfallenden organischen Abfälle. Bislang werden diese sogenannten Nebenströme von Entsorgungsbetrieben abgeholt und kompostiert oder in Vergärungsanlagen verstromt. Nachteilig hieran sind die anfallenden Entsorgungskosten und die energetische Verwertung. Insektenlarven ermöglichen eine Umwandlung von Nebenströmen in hochwertige Futter- oder Nahrungsmittel.

Die Produktion von Insektenlarven geschieht herkömmlicherweise in Niedriglohnländern wenig technisiert. In Hochlohnländern werden die Lohnkosten durch Automatisierung reduziert. Um mit üblichen Futtermitteln konkurrieren zu können, müssen die Anlagen skaliert werden. In Hochlohnländern geplante Fabriken benötigen daher üblicherweise größere Mengen an Nebenströmen als Input, um wirtschaftlich konkurrenzfähig zu sein.

Konventionelle Produktionssysteme verwenden üblicherweise einzelne Mastwannen, in denen die Insektenlarven aufgezogen werden. Die Mastwannen können gestapelt werden oder in einer anderen Weise Teil eines teilautomatisierten Systems sein, sodass eine Befüllung oder Ernte zumindest teilautomatisiert stattfinden kann.

Konventionelle Produktionssysteme für Insektenlarven lassen sich nur schwer oder nicht wirtschaftlich kleiner skalieren, damit sie dezentral am Ort eines anfallenden Nebenstroms aufgestellt werden können. Zudem umfassen konventionelle Produktionssysteme häufig investitionsintensive Anlagen für die Nachverarbeitung der Insektenlarven, da diese sonst nicht lagerfähig sind. Ferner benötigen konventionelle Produktionssysteme häufig Personal für das Behälterhandling. Dadurch können konventionelle Produktionssysteme nicht autark betrieben werden.

Der Erfindung liegt die Aufgabe zugrunde, dem Stand der Technik eine Verbesserung zur Verfügung zu stellen.

Nach einem ersten Aspekt der Erfindung löst die Aufgabe eine Vorrichtung zur Aufzucht und Ernte von Insektenlarven aufweisend zumindest eine Mastwanne, zumindest ein Antriebsmittel und zumindest eine Trägerstruktur, wobei die Mastwanne zumindest einen Boden und zumindest eine Seitendwand aufweist und der Boden und die Seitenwand ein Mastwannenvolumen zumindest teilweise begrenzen und wobei das Antriebsmittel und die zumindest eine Mastwanne in einer Wirkverbindung zueinanderstehen, sodass die zumindest eine Mastwanne von einer ersten Position in eine zweite Position gedreht werden kann, sodass ein Inhalt der Mastwanne in der zweiten Position aus der Mastwanne herausfällt. Dadurch können die Insektenlarven vereinfacht automatisiert geerntet werden.

Die Aufzucht und Ernte von Insektenlarven kann alle Stadien der Insektenlarvenentwicklung von den Eiern der Insekten bis zum Puppen-Stadium der Larve sowie der nachgelagerten Weiterverarbeitung zu Futtermittel oder einem anderen Zwischen- oder Endprodukt umfassen. Insbesondere kann die Aufzucht und Ernte von Insektenlarven das Mästen der Insektenlarven ab dem ersten oder zweiten Larvenstadium sowie die Ernte und Weiterverarbeitung der Insektenlarven zu Futtermittel oder einem anderen Zwischen- oder Endprodukt umfassen.

Bei den Insektenlarven kann es sich um jede beliebige Art von flügellosen Insektenlarven handeln. Vorzugsweise kann es sich bei den Insektenlarven um die Larven der Schwarzen Soldatenfliege (Hermetia Illucens) handeln. Die Larven der Schwarzen Soldatenfliege haben sich als besonders robust und schnell wachsend für eine Vielzahl von verschiedenen Substraten erwiesen. Die Larven der Schwarzen Soldatenfliege können über einen Umgebungstemperaturbereich von größer oder gleich 20 °C bis kleiner oder gleich 40 °C aufgezogen werden. Alternativ oder zusätzlich kann es sich bei den Insektenlarven um die Larven des Mehlkäfers (Tenebrio Molitor) oder die Larven des Glänzendschwarzen Getreideschimmelkäfers (Alphitobius Diaperimus) handeln.

Bei der zumindest einen Mastwanne kann es sich um jegliche für die Aufzucht und Ernte von Insektenlarven geeignete Mastwanne handeln.

Der Boden der Mastwanne weist eine erste Erstreckungsrichtung, eine zweite Erstreckungsrichtung und eine dritte Erstreckungsrichtung auf. Die Erstreckungsrichtungen stehen alle orthogonal aufeinander. Mit anderen Worten kann jeder Punkt im Raum durch eine Angabe der Koordinaten des Punktes entlang der drei Erstreckungsrichtungen beschrieben werden.

Die erste Erstreckungsrichtung kann auch als Länge bezeichnet werden. Die zweite Erstreckungsrichtung kann auch als Breite Bezeichnet werden. Die dritte Erstreckungsrichtung kann auch als Höhe bezeichnet werden.

Der Boden kann in der durch die Länge und die Breite aufgespannten Ebene eine rechteckige Form aufweisen. Eine rechteckige Form im Rahmen der Erfindung weist eine Länge auf, die größer als eine Breite ist. Die Länge kann um größer oder gleich 150 % oder um größer oder gleich 200 % oder um größer oder gleich 300 % oder um größer oder gleich 500 % oder um größer oder gleich 1000 % größer als die Breite sein. Ein so ausgebildetes Bauteil kann auch als rechteckiges Bauteil bezeichnet werden.

Der Boden kann eine Länge von größer oder gleich 4 m oder größer oder gleich 6 m oder größer oder gleich 8 m oder größer oder gleich 12 m aufweisen. Der Boden kann eine Breite von größer oder gleich 20 cm oder größer oder gleich 40 cm oder größer der gleich 60 cm oder größer oder gleich 80 cm oder größer oder gleich 120 cm aufweisen.

Der Boden der Mastwanne kann ein flächiges Bauteil sein. Ein flächiges Bauteil im Rahmen der Erfindung weist eine Höhe auf, die kleiner als die Länge und die Breite ist. Die Höhe kann um größer oder gleich 50 % oder um größer oder gleich 75 % oder um größer oder gleich 90 % oder um größer oder gleich 95 % kleiner als die Länge und/oder die Breite sein.

Der Boden der Mastwanne kann ein ebenes Bauteil sein. Ein ebenes Bauteil im Rahmen der Erfindung weist eine Höhe auf, die konstant über die von der Länge und Breite aufgespannten Fläche verläuft. Ein ebenes Bauteil kann ein Blech sein.

Die zumindest eine Seitenwand kann als rechteckiges Bauteil und/oder als flächiges Bauteil und/oder als ebenes Bauteil ausgebildet sein.

Die zumindest eine Seitenwand kann senkrecht zum Boden der Mastwanne angeordnet sein. Die zumindest eine Seitenwand und der Boden der Mastwanne können einen Winkel von kleiner oder gleich 120 ° oder einen Winkel von kleiner oder gleich 105 ° oder einen Winkel von kleiner oder gleich 90 ° oder einen Winkel von kleiner oder gleich 75 ° einschließen. Dadurch kann verhindert werden, dass die Insektenlarven aus der Mastwanne herausklettern.

Die zumindest eine Seitenwand kann mit dem Boden verbunden sein. Die zumindest eine Seitenwand weist eine senkrechte Komponente zu dem Boden auf, wenn die zumindest eine Seitenwand mit dem Boden verbunden ist.

Der Boden und die zumindest eine Seitenwand der Mastwanne können das Mastwannenvolumen zumindest teilweise begrenzen. Die Mastwanne kann dazu zumindest eine Öffnung aufweisen. Die Öffnung kann verschließbar ausgebildet sein. Die Öffnung kann dem Boden gegenüberliegen. Alternativ oder zusätzlich kann die zumindest eine Seitenwand eine Öffnung aufweisen. Wiederum alternativ oder zusätzlich kann der Boden eine Öffnung aufweisen.

Die Mastwanne kann eine Höhe von größer oder gleich 5 cm oder größer oder gleich 15 cm oder größer oder gleich 30 cm oder größer oder gleich 50 cm aufweisen. Dadurch kann verbessert verhindert werden, dass die Insektenlarven aus der Mastwanne herausklettern. Je höher die Mastwanne und je geringer der Winkel zwischen den Seitenwänden und dem Boden der Mastwanne desto besser kann verhindert werden, dass die Insektenlarven aus der Mastwanne herausklettern.

Im bestimmungsgemäßen Gebrauch kann die Öffnung der Mastwanne in der ersten Position nach oben zeigen. Dadurch kann der Inhalt der Mastwanne in der Mastwanne aufgenommen werden und kann nicht aus der Mastwanne herausfallen. Mit anderen Worten ist oben die Richtung, die im bestimmungsgemäßen Gebrauch der Mastwanne der wirkenden Schwerkraft entgegengesetzt ist.

Die Öffnung der Mastwanne kann in der zweiten Position nach unten zeigen. Dadurch fällt der Inhalt der Mastwanne aus der Mastwanne durch die wirkende Schwerkraft heraus. Mit anderen Worten ist unten die Richtung, die im bestimmungsgemäßen Gebrauch der Mastwanne in derselben Richtung wirkt wie die wirkende Schwerkraft.

Die Mastwanne kann zumindest drei, vier oder mehr als vier Seitenwände aufweisen. Die Seitenwände können Mastwannenvolumen in Richtung der Länge und der Breite des Bodens begrenzen.

Nach einer bevorzugten Ausführungsform kann die Mastwanne vier Seitenwände aufweisen, wobei zwei Seitenwände das Mastwannenvolumen in Richtung der Länge des Bodens begrenzen und zwei Seitenwände das Mastwannenvolumen in Richtung der Breite des Bodens begrenzen. Mit anderen Worten ist eine so ausgebildete Mastwanne quaderförmig. Dadurch kann die Mastwanne besonders einfach als Schweißbauteil oder Blechbiegebauteil hergestellt werden und damit besonders kostengünstig gefertigt werden.

Alternativ kann die Mastwanne eine einzelne, zusammenhängende um den Boden umlaufende Seitenwand aufweisen die das Mastwannenvolumen zu den Seiten begrenzt.

Die zweite Position der Mastwanne ist um einen Winkel größer oder gleich 135° zu der ersten Position versetzt sein, vorzugsweise größer oder gleich 180°. Bei den Winkelangaben kann es sich um vorzeichenneutrale, betragliche Winkelangaben handeln.

Die Mastwanne kann eine Länge aufweisen, die in derselben Richtung wie die Länge des Bodens verläuft. Die Mastwanne kann eine Breite aufweisen, die in derselben Richtung wie die Breite des Bodens verläuft. Die Mastwanne kann eine Höhe aufweisen, die in derselben Richtung wie die Höhe des Bodens verläuft. Die erste Erstreckungsrichtung und die zweite Erstreckungsrichtung und die dritte Erstreckungsrichtung der Mastwanne stehen orthogonal aufeinander.

Die Mastwanne weist eine Drehachse auf um welche die Mastwanne von der ersten Position in die zweite Position gedreht werden kann. Die Längsachse ist die Symmetrieachse der Mastwanne in der Richtung der Länge der Mastwanne. Dadurch weist die Mastwanne ein besonders geringes Trägheitsmoment bezogen auf Drehungen um die Drehachse auf. Mit anderen Worten kann die Mastwanne dadurch mit einem besonders geringen Drehmoment gedreht werden.

Die Drehachse der Mastwanne und die Längsachse der Mastwanne verlaufen koaxial zueinander. Dadurch kann die Mastwanne mit einem nochmals geringeren Drehmoment gedreht werden.

Die Breitenachse ist die Symmetrieachse der Mastwanne in der Richtung der Breite der Mastwanne. Gemäß einer nicht erfindungsgemäßen Ausführungsform kann die Drehachse der Mastwanne parallel zur Breitenachse der Mastwanne verlaufen. Gemäß dieser Ausführungsform kann die Drehachse der Mastwanne kollinear zu der Breitenachse der Mastwanne verlaufen. Gemäß dieser Ausführungsform kann die Drehachse der Mastwanne koaxial zu der Breitenachse der Mastwanne verlaufen.

Der orthogonal zur Länge der Mastwanne liegende Querschnitt der Mastwanne kann zumindest abschnittweise rechteckig ausgebildet sein. Alternativ oder zusätzlich kann der orthogonal zur Länge der Mastwanne liegende Querschnitt der Mastwanne zumindest abschnittweise halbkreisförmig ausgebildet sein.

Die Mastwanne kann abschnittweise oder vollständig aus Holz gebildet sein. Dadurch kann die Feuchtigkeit des Inhalts der Mastwannen besser reguliert werden und die Gefahr für Schimmelbildung kann reduziert werden.

Die Mastwanne kann abschnittweise oder vollständig aus einem Kunststoff gebildet sein. Der Kunststoff kann ein faserverstärkter Kunststoff sein. Der Kunststoff kann mit Glasfasern und/oder Karbonfasern verstärkt sein. Der Kunststoff kann jeder beliebige Kunststoff sein, insbesondere PP, PA, ABS, PMMA, PU, PE.

Die Mastwanne kann als ein Spritzgießbauteil oder als ein Thermoformbauteil ausgebildet sein. Dadurch kann die Mastwanne in einem Fertigungsschritt hergestellt. Damit können die Herstellungskosten reduziert werden.

Die Mastwanne kann abschnittweise oder vollständig aus einem metallischen Werkstoff gebildet sein. Der metallische Werkstoff kann eine Stahllegierung oder eine Aluminiumlegierung sein. Alternativ kann der metallische Werkstoff jeder beliebige metallische Werkstoff sein.

Die Mastwanne kann als Blechteil, insbesondere als aus einem Blech geformtes Blechbiegeteil, ausgebildet sein. Die Mastwanne kann einen zur Länge der Mastwanne orthogonal liegenden Querschnitt aufweisen, der aus einem Blech halbkreisförmig geformt werden kann. Dadurch kann die Mastwanne nochmals vereinfacht hergestellt werden.

Alternativ oder zusätzlich kann die Mastwanne als Schweißbauteil ausgebildet sein.

Die Mastwanne kann zumindest einen Aufnahmedom zur Aufnahme der Mastwanne in der Trägerstruktur aufweisen.

Der Aufnahmedom kann an einer Seitenwand angeordnet sein. Der Aufnahmedom kann an einer Seitenwand angeordnet sein, welche die Länge des Bodens begrenzt.

Der Aufnahmedom kann sich von der Seitenwand von dem Mastwannenvolumen weg erstrecken. Der Aufnahmedom kann sich von der Seitenwand orthogonal nach außen erstrecken.

Der Aufnahmedom kann mit der Seitenwand verschweißt, verklebt, vernietet, verschraubt oder in sonstiger Weise lösbar oder nicht lösbar verbunden sein. Der Aufnahmedom kann einen Flansch aufweisen, wobei der Flansch mit der Seitenwand mit Schrauben verbunden ist. Die Schrauben des Flansches können dabei in einem Kreis um den Aufnahmedom angeordnet sein, wobei die Rotationsachse des Aufnahmedoms und des Kreises identisch sein können.

Der Aufnahmedom kann parallel oder kollinear oder koaxial zur Längsachse der Mastwanne angeordnet sein. Alternativ kann der Aufnahmedom parallel oder kollinear oder koaxial zur Breitenachse der Mastwanne angeordnet sein.

Der Aufnahmedom kann als Pin, als Zapfen, als Welle oder als sonstiger zylindrischer Körper ausgebildet sein.

Die Mastwanne kann zwei oder mehr Aufnahmedome aufweisen. Die Mastwanne kann zwei Aufnahmedome aufweisen, die jeweils an den sich gegenüberliegenden, die Länge des Bodens begrenzenden Seitenwänden angeordnet sind. Beide Aufnahmedome können parallel oder kollinear oder koaxial zur Längsachse angeordnet sein.

Die Mastwanne kann zumindest eine Verstärkungsstruktur aufweisen, wobei die Verstärkungsstruktur mit dem Boden und/oder zumindest einer Seitenwand verbunden sein kann. Eine derart ausgebildete Mastwanne weist den Vorteil auf, dass die Mastwanne eine erhöhte Stabilität aufweist. Denn bei langen Mastwannen kann durch die Verstärkungsstruktur ein Durchhängen der Mastwanne in der Trägerstruktur vermieden werden. Weiterhin kann die Stabilität der Mastwanne beim Drehen von der ersten Position in die zweite Position erhöht werden.

Die Verstärkungsstruktur kann als rechteckiges Bauteil ausgebildet sein. Die Verstärkungsstruktur kann als flächiges Bauteil ausgebildet sein. Alternativ kann die Verstärkungsstruktur als Strebe, als Stab, als Rundstab, als Leiste, als T-Träger oder als Doppel-T-Träger ausgebildet sein.

Die Verstärkungsstruktur kann abschnittweise oder vollständig aus einem Metall und/oder einem Kunststoff gebildet sein. Die Verstärkungsstruktur kann die gleichen Werkstoffe wie die Mastwanne aufweisen.

Die Verstärkungsstruktur kann zumindest abschnittweise in Längsrichtung der Mastwanne verlaufen. Alternativ kann die Richtung der Verstärkungsstruktur eine Parallelkomponente mit der Längsrichtung der Mastwanne aufweisen. Alternativ kann die Verstärkungsstruktur in Breitenrichtung der Mastwanne verlaufen. Die Verstärkungsstruktur kann zumindest abschnittweise in Längsrichtung und Breitenrichtung der Mastwanne verlaufen. Mit anderen Worten kann die Verstärkungsstruktur zumindest abschnittweise diagonal entlang der Länge und der Breite der Mastwanne verlaufen.

Die Verstärkungsstruktur kann mit dem Boden und/oder der zumindest einen Seitenwand verschweißt und/oder verschraubt und/oder vernietet und/oder verklebt und/oder verpresst und/oder in sonstiger Weise lösbar oder nicht lösbar verbunden sein.

Alternativ kann die Verstärkungsstruktur monolithisch mit dem Boden und/oder der zumindest einen Seitenwand verbunden sein.

Ein Bauteil, das monolithisch mit einem anderen Bauteil verbunden ist, ist aus einem Stück hergestellt und insbesondere fugenlos verbunden.

Die Verstärkungsstruktur kann unter dem Boden der Mastwanne angeordnet sein. Insbesondere kann die Verstärkungsstruktur derart unter dem Boden der Mastwanne angeordnet sein, dass die Hüllkurve der Drehung der Mastwanne durch die Verstärkungsstruktur nicht vergrößert wird. Mit anderen Worten kann die Verstärkungsstruktur so unter dem Boden der Mastwanne angeordnet sein, dass die Summe der Höhe der Mastwanne und der Höhe der Verstärkungsstruktur kleiner oder gleich der Breite der Mastwanne ist. Dadurch können die Mastwannen dichter in vertikalen Reihen zueinander angeordnet werden.

Alternativ oder zusätzlich können zwei oder mehr Verstärkungsstrukturen unter dem Boden der Mastwanne angeordnet werden. Die Verstärkungsstrukturen können parallel zueinander angeordnet sein. Die Verstärkungsstrukturen können in Längsrichtung der Mastwanne verlaufen.

Die Verstärkungsstruktur kann in dem Mastwannenvolumen angeordnet sein und das Mastwannenvolumen zumindest in ein erstes Untervolumen und in ein zweites Untervolumen unterteilen. Dadurch kann die Stabilität der Mastwanne, insbesondere bei einer Drehung der Mastwanne, erhöht werden.

Die Verstärkungsstruktur kann in Längsrichtung der Mastwanne verlaufen und das Mastwannenvolumen entlang der Länge der Mastwanne in zwei gleich große Untervolumina, ein erstes Untervolumen und ein zweites Untervolumen, unterteilen. Dadurch können beide Untervolumina gleichmäßig mit Substrat befüllt werden. Die Mastwanne kann eine beliebige Anzahl weiterer Verstärkungsstrukturen aufweisen, die in Längsrichtung der Mastwanne verlaufen und das Mastwannenvolumen entlang der Länge in beliebig viele weitere Untervolumina unterteilt. Die Untervolumina können alle gleich groß sein.

Die Verstärkungsstruktur kann in Breitenrichtung der Mastwanne verlaufen und das Mastwannenvolumen entlang Breitenrichtung der Mastwanne in zwei gleich große Untervolumina, ein erstes und eine zweites Untervolumen, unterteilen. Dadurch kann die Stabilität beim Drehen der Mastwanne erhöht werden. Die Mastwanne kann eine beliebige Anzahl weiterer Verstärkungsstrukturen aufweisen, die in Breitenrichtung der Mastwanne verlaufen und das Mastwannenvolumen entlang der Breite der Mastwanne in beliebig viele weitere Untervolumina unterteilt. Die Untervolumina können alle gleich groß oder unterschiedlich groß sein.

Die Mastwanne kann eine Verstärkungsstruktur aufweisen, die in Längsrichtung der Mastwanne verläuft und eine Verstärkungsstruktur, die in Breitenrichtung der Mastwanne verläuft. Dadurch kann das Mastwannenvolumen in vier rechteckige Untervolumina unterteilt werden, die gleich groß sein können. Dadurch kann die Stabilität der Mastwanne beim Drehen nochmals weiter erhöht werden. Die Mastwanne kann beliebig viele Verstärkungsstrukturen aufweisen, die in Längsrichtung verlaufen und beliebig viele Verstärkungsstrukturen aufweisen, die in Breitenrichtung verlaufen. Dadurch kann das Mastwannenvolumen in beliebig viele rechteckige Untervolumina unterteilt werden.

Die Trägerstruktur kann als Metallkonstruktion ausgebildet sein. Insbesondere kann die Trägerstruktur als Schweißkonstruktion ausgebildet sein. Die Trägerstruktur kann weitere Verbindungsmittel wie Schrauben, Niete, Bolzen oder sonstige weitere Verbindungsmittel aufweisen.

Die Trägerstruktur kann zumindest eine Vorderwand und/oder eine Rückwand zur Aufnahme weiterer Komponenten aufweisen. Weitere Komponenten können Antriebsmittel, Aufnahmeeinrichtung, Belüftungsöffnungen, Befüllöffnungen oder sonstige Komponenten sein.

Die Vorderwand kann eine Vorderseite und eine Rückseite aufweisen. Die Rückwand kann eine Vorderseite und eine Rückseite aufweisen. Bei bestimmungsgemäßem Gebrauch liegt die Rückseite der Vorderwand der Rückseite der Rückwand gegenüber.

Die zumindest eine Mastwanne kann in der Trägerstruktur zwischen der Vorderwand und der Rückwand aufgenommen sein. Dadurch kann die Stabilität der Vorrichtung erhöht werden.

Die zumindest eine Mastwanne kann in der Trägerstruktur zwischen der Vorderwand und der Rückwand aufgenommen sein, sodass die Rückseite der Vorderwand und die Rückseite der Rückwand sich gegenüberliegen und der Mastwanne zugewandt sind. Mit anderen Worten ist die Rückseite der Vorderwand und die Rückseite der Rückwand der Mastwanne zugewandt, wenn die Mastwanne zwischen der Vorderwand und der Rückwand in der Trägerstruktur aufgenommen ist.

Die Vorderwand und/oder die Rückwand kann zumindest eine Befüllöffnung aufweisen, durch welche die zumindest eine Mastwanne mit Substrat und/oder Insektenlarven befüllt werden kann. Die Vorderwand und/oder die Rückwand kann genauso viele Befüllöffnungen wie Mastwannen aufweisen. Die Befüllöffnungen können so angeordnet sein, dass jeweils eine Befüllöffnung einer Mastwanne zugeordnet ist. Eine Befüllöffnung ist einer Mastwanne zugeordnet, wenn die Mastwanne durch diese Befüllöffnung mit Substrat und/oder Insektenlarven befüllt werden kann. Die Befüllöffnung kann mittig bezogen auf die Breitenrichtung über der Mastwanne angeordnet sein.

Alternativ oder zusätzlich kann die Vorderwand und/oder die Rückwand doppelt so viele Befüllöffnungen wie Mastwannen aufweisen. Die Befüllöffnungen können so angeordnet sein, dass jeweils zwei Befüllöffnungen einer Mastwanne zugeordnet sind. Die zwei Befüllöffnungen können beabstandet zueinander angeordnet sein. Der Abstand zwischen den beiden Befüllöffnungen kann kleiner oder gleich oder größer als der Abstand einer Befüllöffnung zu einer Seitenwand einer Mastwanne sein. Die zwei Befüllöffnungen können so bezogen auf die Breitenrichtung über der Mastwanne angeordnet sein, dass der Mittelpunkt des Abstandes zwischen den beiden Befüllöffnungen mittig über der Mastwanne angeordnet ist.

Die Befüllöffnung kann einen elliptischen oder kreisrunden Querschnitt aufweisen. Dadurch kann die der Befüllöffnung zugeordneten Mastwannen mit geringerem Druck befüllt werden. Alternativ kann die Befüllöffnung einen rechteckigen oder einen quadratischen Querschnitt aufweisen.

Die Befüllöffnung kann ein Anschlusselement zum Anschluss an eine Zuführeinrichtung und/oder an eine Befülleinrichtung aufweisen. Das Anschlusselement kann an der Vorderseite der Vorderwand und/oder an der Vorderseite der Rückwand angeordnet sein. Das Anschlusselement kann sich von der Vorderseite der Vorderwand und/oder von der Vorderseite der Rückwand erstrecken, insbesondere orthogonal erstrecken. Dadurch kann eine Befülleinrichtung und/oder eine Zuführeinrichtung vereinfacht angeschlossen werden.

Das Anschlusselement kann als Rohr, als Schlauch oder als Stutzen ausgebildet sein.

Die Befüllöffnung kann ein Abgabeelement zur Abgabe von Substrat und/oder Insektenlarven in eine Mastwanne aufweisen. Das Abgabeelement kann an der Rückseite der Vorderwand und/oder an der Rückseite der Rückwand angeordnet sein. Das Abgabeelement kann sich von der Rückseite der Vorderwand und/oder von der Rückseite der Rückwand erstrecken, insbesondere orthogonal erstrecken.

Das Abgabeelement kann sich von der Rückseite der Vorderwand und/oder von der Rückseite der Rückwand bis über das Mastwannenvolumen erstrecken, sodass Insektenlarven und/oder Substrat von dem Abgabeelement in die zugeordnete Mastwanne abgegeben können, wenn die zumindest eine Mastwanne in der ersten Position zwischen der Vorderwand und der Rückwand der Trägerstruktur aufgenommen ist.

Das Abgabeelement kann als Rohr, als Schlauch oder als Stutzen ausgebildet sein.

Das Anschlusselement und das Abgabeelement können als ein monolithisch verbundenes Befüllelement ausgebildet sein. Das Befüllelement kann in der Vorderwand und/oder der Rückwand aufgenommen sein. Das Befüllelement kann mittig in der Vorderwand und/oder der Rückwand aufgenommen sein, sodass sich das Befüllelement ausgehend von der Vorderwand und/oder Rückwand orthogonal zur Vorderseite und Rückseite erstreckt, insbesondere gleichmäßig erstreckt.

Die Vorderwand und/oder die Rückwand können eine Belüftungsöffnung zur Belüftung der Mastwannen aufweisen. Die Vorderwand und/oder die Rückwand können zwei oder mehr Belüftungsöffnungen zur Belüftung der Mastwannen aufweisen.

Der Querschnitt einer Belüftungsöffnung der Vorderwand kann den Querschnitt genau einer gegenüberliegenden Belüftungsöffnung der Rückwand teilweise oder vollständig überdecken. Diese zwei Belüftungsöffnungen bilden ein korrespondierendes Paar von Belüftungsöffnungen. Dadurch kann ein kontrollierter Luftstrom über die Mastwannen erreicht werden.

Die Vorrichtung kann eine Vielzahl von korrespondierenden Paaren von Belüftungsöffnungen aufweisen.

Alternativ kann der Querschnitt einer Belüftungsöffnung der Vorderwand den Querschnitt einer oder mehrerer gegenüberliegender Belüftungsöffnungen der Rückwand teilweise oder vollständig überdecken. Dadurch kann die Trägerstruktur vereinfach hergestellt werden. Alternativ kann der Querschnitt einer Belüftungsöffnung der Rückwand den Querschnitt einer oder mehrerer gegenüberliegender Belüftungsöffnungen der Vorderwand teilweise oder vollständig überdecken.

Die Belüftungsöffnungen der Vorderwand und/oder der Rückwand können oberhalb der Seitenwände einer Mastwanne in der ersten Position angeordnet sein, wenn die Mastwanne in der Trägerstruktur aufgenommen ist. Mit anderen Worten kann der Querschnitt der Belüftungsöffnungen nicht von der Mastwanne in der ersten Position überdeckt werden, wenn die Mastwanne in der Trägerstruktur aufgenommen ist. Dadurch kann ein kontinuierlicher Luftstrom über den Mastwannen erreicht werden.

Die Belüftungsöffnung kann einen elliptischen oder kreisrunden oder rechteckigen oder einen quadratischen Querschnitt aufweisen. Alternativ kann die Belüftungsöffnung jeden beliebigen Querschnitt aufweisen.

Vorzugsweise ist eine Vielzahl von Belüftungsöffnungen in der Vorderwand und/oder der Rückwand angeordnet. Eine Vielzahl kann bezogen auf die Anzahl der Mastwannen angegeben werden. Eine Vielzahl kann zwei oder mehr Belüftungsöffnungen je Mastwanne sein, oder drei oder mehr Belüftungsöffnungen je Mastwanne oder vier oder mehr Belüftungsöffnungen je Mastwanne oder fünf oder mehr Belüftungsöffnungen je Mastwanne. Dadurch kann ein vergrößerter Luftstrom über die Mastwanne geführt werden.

Die Belüftungsöffnung kann eine Filtereinrichtung aufweisen. Die Filtereinrichtung kann in der Belüftungsöffnung angeordnet sein. Die Filtereinrichtung ist dazu eingerichtet, Störstoffe und Störinsekten aus der Umgebungsluft zu filtern, sodass der über die Mastwanne geleitete Luftstrom frei von Störstoffen und Störinsekten ist. Dadurch können die Aufzucht und Ernte der Insektenlarven verbessert werden.

Das Antriebsmittel kann einen elektrischen Motor und/oder ein Getriebe aufweisen. Das Antriebsmittel kann ein Kopplungselement aufweisen, mit dem eine Wirkverbindung, insbesondere eine kinematische Wirkverbindung, zwischen dem Antriebsmittel und der Mastwanne hergestellt werden kann. Das Kopplungselement kann als Riemenscheibe und/oder ein Kettenrad und/oder sonstiges Kopplungselement ausgebildet sein.

Das Antriebsmittel kann in der Trägerstruktur und/oder an der Mastwanne aufgenommen sein. Das Antriebsmittel kann derart an der Trägerstruktur aufgenommen sein, dass es die Mastwanne direkt um die Drehachse drehen kann. Mit anderen Worten kann das Antriebsmittel so an der Trägerstruktur aufgenommen sein, dass die Drehachse des elektrischen Motors des Antriebsmittels koaxial zu der Drehachse der Mastwanne angeordnet ist. Dadurch kann die Mastwanne direkt und ohne ein Getriebe zwischen Motor und Mastwanne gedreht werden. Dadurch kann die Vorrichtung kostengünstiger hergestellt werden.

Der elektrische Motor des Antriebsmittels kann ein permanenterregter elektrischer Motor sein. Dadurch kann die Mastwanne ohne zusätzliche weitere Bremse in einer beliebigen Winkelposition gehalten werden.

Insbesondere kann das Antriebsmittel ein Getriebe aufweisen. Das Getriebe kann als Schneckengetriebe ausgebildet sein. Dadurch kann die Mastwanne mit einer konstanten und nahezu beschleunigungsfreien Winkelgeschwindigkeit von der ersten Position in die zweite Position gedreht werden. Dadurch können die Insektenlarven besonders schonend geerntet werden.

Besonders bevorzugt kann das Schneckengetriebe als selbsthemmendes Schneckengetriebe ausgebildet sein. Dadurch kann die Mastwanne in jede beliebige Winkelposition ohne eine zusätzliche Motorbremse gedreht und gehalten werden.

Das Antriebsmittel kann als Handkurbel ausgebildet sein. Dadurch kann die Mastwanne stromlos von der ersten Position in die zweite Position gedreht werden. Dadurch kann die Vorrichtung besonders kostengünstig hergestellt werden.

Zumindest eine Mastwanne kann in der Trägerstruktur aufgenommen sein. Dadurch kann die Stabilität der Vorrichtung erhöht werden.

Die zumindest eine Mastwanne kann in der Trägerstruktur zwischen der Vorderwand und der Rückwand aufgenommen sein. Insbesondere können alle Mastwannen in der Trägerstruktur zwischen der Vorderwand und der Rückwand aufgenommen sein. Dadurch kann die Stabilität der Vorrichtung erhöht werden und gleichzeitig ein definierter Luftstrom über jede Mastwanne geführt werden.

Die Vorrichtung kann zumindest eine Aufnahmeeinrichtung aufweisen, wobei die zumindest eine Mastwanne über die zumindest eine Aufnahmeeinrichtung in der Trägerstruktur aufgenommen sein kann. Dadurch kann die Mastwanne besonders stabil gedreht werden.

Die zumindest eine Aufnahmeeinrichtung kann in der Vorderwand und/oder der Rückwand der Trägerstruktur angeordnet sein.

Die Aufnahmeeinrichtung kann als zylindrische Aussparung in der Vorderwand und/oder der Rückwand der Trägerstruktur ausgebildet sein.

Die Aufnahmeeinrichtung kann den Aufnahmedom der Mastwanne aufnehmen, sodass die Mastwanne in der Trägerstruktur aufgenommen werden kann. Die Mastwanne kann durch den Aufnahmedom drehbar in der Aufnahmeeinrichtung der Trägerstruktur aufgenommen werden.

Die Aufnahmeeinrichtung kann ein Lager aufweisen, durch welches die Mastwanne drehbar in der Trägerstruktur aufgenommen werden kann.

Der Aufnahmedom kann durch die Aufnahmeeinrichtung hindurch ragen, wenn die Mastwanne in der Trägerstruktur aufgenommen ist. Mit anderen Worten kann der Aufnahmedom von der Rückseite der Vorderwand und/oder der Rückseite der Rückwand durch die Aufnahmeeinrichtung in der Vorderwand und/oder der Rückwand hindurch ragen, wenn die Mastwanne in der Trägerstruktur aufgenommen ist. An dem durch die Vorderwand und/oder Rückwand hindurchragenden Abschnitt des Aufnahmedoms kann ein Kopplungselement zur kinematischen Kopplung einer Rotationsbewegung angeordnet sein.

Ein Kopplungselement kann als Riemenscheibe, Kettenrad oder ein sonstiges Element zur kinematischen Kopplung einer Rotationsbewegung ausgebildet sein.

Der durch die Vorderwand und/oder Rückwand hindurchragende Abschnitt des Aufnahmedoms kann als freier Abschnitt des Aufnahmedoms bezeichnet werden.

An dem freien Abschnitt des Aufnahmedoms können zwei oder mehr Kopplungselemente angeordnet sein. An dem freien Abschnitt des Aufnahmedoms können zwei oder mehr Kopplungselemente koaxial zum Aufnahmedom mit einem definierten Abstand zueinander angeordnet sein. Insbesondere können zwei oder mehr Kettenräder koaxial zum Aufnahmedom mit einem definierten Abstand zueinander auf dem Aufnahmedom angeordnet sein.

Die Vorrichtung kann zumindest zwei Aufnahmeeinrichtungen aufweisen, wobei die zumindest eine Mastwanne über die zumindest zwei Aufnahmeeinrichtungen in der Trägerstruktur aufgenommen sein kann.

Die zumindest zwei Aufnahmeeinrichtungen können in der Vorderwand und/oder der Rückwand der Trägerstruktur angeordnet sein.

Insbesondere kann eine Aufnahmeeinrichtung in der Vorderwand angeordnet sein und eine Aufnahmeeinrichtung in der Rückwand angeordnet sein, sodass die beiden Aufnahmeeinrichtungen koaxial zueinander angeordnet sind und sich gegenüberliegen und die Mastwanne zwischen Vorderwand und Rückwand in der Trägerstruktur aufgenommen werden kann. Dadurch kann die Mastwanne nochmals kontrollierter und stabiler gedreht werden.

Die Vorrichtung kann doppelt so viele Aufnahmeeinrichtungen wie Mastwannen aufweisen, wobei jeweils eine Mastwanne über jeweils zwei Aufnahmeeinrichtungen in der Trägerstruktur aufgenommen sein kann.

Der elektrische Motor kann so an der Vorderwand und/oder der Rückwand der Trägerstruktur angeordnet sein, dass der elektrische Motor direkt mit dem freien Abschnitt des Aufnahmedoms der Mastwanne verbunden sein kann, sodass der elektrische Motor die Mastwanne direkt von der ersten Position in die zweite Position drehen kann. Mit anderen Worten kann die Drehachse des elektrischen Motors koaxial zum Aufnahmedom verlaufen.

Alternativ kann das Getriebe des Antriebsmittels so an der Vorderwand und/oder der Rückwand der Trägerstruktur angeordnet sein, dass das Getriebe mit dem freien Abschnitt des Aufnahmedoms der Mastwanne verbunden sein kann, sodass der elektrische Motor die Mastwanne über das Getriebe von der ersten Position in die zweite Position drehen kann.

Die Trägerstruktur kann ein oder mehrere Kragarmregale aufweisen. Die zumindest eine Aufnahmeeinrichtung kann als ein Kragarm eines Kragarmregals der Trägerstruktur ausgebildet sein. Die Kragarmregale können jeweils eine Vielzahl von Kragarmen aufweisen.

Die Kragarme des Kragarmregals weisen eine Erstreckung auf, die größer oder gleich der Breite von zwei Mastwannen ist. Mit anderen Worten können zumindest zwei Mastwannen nebeneinander auf den Kragarmen angeordnet werden.

Die zumindest eine Mastwanne kann in der Trägerstruktur herausnehmbar angeordnet sein. Vorzugsweise liegt die zumindest eine Mastwanne auf zumindest zwei Kragarmen eines Kragarmregals der Trägerstruktur auf. Vorzugsweise liegt die Mastwanne mit dem Boden der Mastwanne auf den zumindest zwei Kragarmen des Kragarmregals der Trägerstruktur auf.

Die Vorrichtung kann zwei oder mehr Mastwannen aufweisen. Dadurch kann die Aufnahmemenge an Insektenlarven in der Vorrichtung erhöht werden.

Die Vorrichtung kann vier oder mehr Mastwannen aufweisen oder zwölf oder mehr Mastwannen oder achtzehn oder mehr Mastwannen. Nach eine bevorzugten Ausführungsform kann die Vorrichtung 24 oder mehr Mastwannen oder 28 oder mehr Mastwannen aufweisen. Dadurch kann die Aufnahmemenge an Insektenlarven in der Vorrichtung nochmals weiter erhöht werden.

Die Mastwannen können alle gleich ausgebildet sein oder unterschiedlich. Beispielsweise können die Mastwannen in einer horizontalen Reihe einen zur Länge der Mastwanne orthogonalen rechteckigen Querschnitt aufweisen. Die Mastwannen der darunter oder darüber liegenden horizontalen Reihe können einen zur Länge der Mastwanne orthogonalen halbkreisförmigen Querschnitt aufweisen. Alternativ oder zusätzlich können die Mastwannen in einer horizontalen Reihe einen zur Länge der Mastwanne orthogonalen Querschnitt aufweisen.

Die Vorrichtung kann eine Verfahreinrichtung aufweisen, wobei die Verfahreinrichtung dazu ausgebildet ist, die zumindest eine Mastwanne in horizontaler und/oder vertikaler Richtung translatorisch zu bewegen.

Die Verfahreinrichtung kann dazu ausgebildet sein, die zumindest eine Mastwanne von einer ersten Mastwannenposition in eine zweite Mastwannenposition in horizontaler und/oder vertikaler Richtung translatorisch zu bewegen.

Die Verfahreinrichtung kann mit der Trägerstruktur verbunden sein und/oder Bestandteil der Trägerstruktur sein.

Die Verfahreinrichtung kann einen ersten vertikal verfahrbaren horizontal verlaufenden Balken und einen dem ersten Balken gegenüberliegenden zweiten vertikal verfahrbaren horizontal verlaufenden Balken aufweisen. Der erste Balken und der zweite Balken sind vorzugsweise bezogen auf die Längsachse einer Mastwanne gegenüberliegend angeordnet. Mit anderen Worten liegen sich der erste Balken und der zweite Balken vorzugsweise derart gegenüber, dass die Mastwanne zwischen dem ersten Balken und dem zweiten Balken angeordnet ist und der erste Balken vom zweiten Balken in Richtung der Längsachse der Mastwanne beabstandet ist.

Alternativ kann die Verfahreinrichtung einen ersten horizontal verfahrbaren vertikal verlaufenden Balken und einen dem ersten Balken gegenüberliegenden zweiten horizontal verfahrbaren vertikal verlaufenden Balken aufweisen. Der erste Balken und der zweite Balken sind vorzugsweise bezogen auf die Längsachse einer Mastwanne gegenüberliegend angeordnet. Mit anderen Worten liegen sich der erste Balken und der zweite Balken vorzugsweise derart gegenüber, dass die Mastwanne zwischen dem ersten Balken und dem zweiten Balken angeordnet ist und der erste Balken vom zweiten Balken in Richtung der Längsachse der Mastwanne beabstandet ist.

Die Verfahreinrichtung kann ein oder mehrere Verfahrmittel zum Verfahren des ersten Balkens und/oder des zweiten Balkens aufweisen. Das Verfahrmittel kann einen Elektromotor aufweisen. Der Elektromotor kann als Direktantrieb für den ersten Balken und/oder den zweiten Balken ausgebildet sein. Alternativ kann der Elektromotor einen Kettenantrieb antreiben.

Der erste Balken und der zweite Balken sind vorzugsweise bewegungsgekoppelt zueinander. Mit anderen Worten können der erste Balken und der zweiten Balken nur gleichzeitig vertikal und/oder horizontal verfahren werden. Ferner befinden sich der erste Balken und der zweite Balken zu jeder Zeit an der gleichen vertikalen und/oder horizontalen Position.

Der erste Balken kann einen ersten Verfahrschlitten aufweisen, der entlang des ersten Balkens horizontal und/oder vertikal verfahrbar ist. Der erste Verfahrschlitten kann eine erste Wannenaufnahmeeinrichtung aufweisen.

Der zweite Balken kann einen zweiten Verfahrschlitten aufweisen, der entlang des zweiten Balkens horizontal und/oder vertikal verfahrbar ist. Der zweite Verfahrschlitten kann eine zweite Wannenaufnahmeeinrichtung aufweisen.

Der erste Verfahrschlitten und der zweite Verfahrschlitten sind vorzugsweise bewegungsgekoppelt zueinander. Mit anderen Worten können der erste Verfahrschlitten und der zweite Verfahrschlitten nur gleichzeitig verfahren werden. Ferner befinden sich der erste Verfahrschlitten und der zweite Verfahrschlitten zu jeder Zeit an der gleichen vertikalen und/oder horizontalen Position.

Die Verfahreinrichtung kann als Portalkran oder als Verfahreinrichtung eines automatischen Hochregallagers ausgebildet sein.

Die Verfahreinrichtung ist dazu ausgebildet, eine Mastwanne aus einer ersten Wannenposition in eine zweite Wannenposition translatorisch zu verfahren. Dazu können der erste Verfahrschlitten und der zweite Verfahrschlitten und/oder der erste Balken und der zweite Balken horizontal und/oder vertikal derart verfahren werden, dass die Mastwanne in der ersten Wannenaufnahmeeinrichtung des ersten Verfahrschlittens und in der zweiten Wannenaufnahmeeinrichtung des zweiten Verfahrschlittens aufgenommen ist.

Die Mastwanne weist vorzugsweise einen ersten Aufnahmedom und einen zweiten Aufnahmedom auf.

Die zumindest eine Mastwanne ist vorzugsweise mittels dem Aufnahmedom in der Verfahreinrichtung aufnehmbar. Die zumindest eine Mastwanne ist vorzugsweise derart in der Verfahreinrichtung aufnehmbar, dass ein erster Aufnahmedom der Mastwanne in der ersten Wannenaufnahmeeinrichtung des ersten Verfahrschlittens der Verfahreinrichtung aufgenommen ist und ein zweiter Aufnahmedom der Mastwanne in der zweiten Wannenaufnahmeeinrichtung des zweiten Verfahrschlittens aufgenommen ist. Eine derart in der Aufnahmeeinrichtung aufgenommene Mastwanne kann durch Verfahren des ersten und zweiten Verfahrschlittens in horizontale und/oder vertikale Richtung und/oder durch Verfahren des ersten und zweiten Balkens in horizontale und/oder vertikale Richtung von einer ersten Wannenposition in eine zweite Wannenposition translatorisch verfahren werden.

In bestimmungsgemäßen Gebrauch der Vorrichtung wird der erste Verfahrschlitten derart horizontal und/oder vertikal verfahren, dass die erste Wannenaufnahmeeinrichtung in horizontaler Richtung an der gleichen Stelle wie der erste Aufnahmedom der Mastwanne angeordnet ist und in vertikaler Richtung unterhalb des ersten Aufnahmedoms der Mastwanne angeordnet ist. Der zweite Verfahrschlitten wird derart horizontal und/oder vertikal verfahren, dass die zweite Wannenaufnahmeeinrichtung in horizontaler Richtung an der gleichen Stelle wie der zweite Aufnahmedom der Mastwanne angeordnet ist und in vertikaler Richtung unterhalb des zweiten Aufnahmedoms der Mastwanne angeordnet ist. Durch Verfahren des ersten Verfahrschlittens und des zweiten Verfahrschlittens und/oder des ersten Balkens und des zweiten Balkens in vertikaler Richtung wird die erste Wannenaufnahmeeinrichtung mit dem ersten Aufnahmedom und die zweite Wannenaufnahmeeinrichtung mit dem zweiten Aufnahmedom in Eingriff gebracht, sodass die Mastwanne in der Verfahreinrichtung aufgenommen ist und von den Kragarmen des Kragarmregals hochgehoben wird, sodass die Mastwanne in vertikaler und/oder horizontaler Richtung durch die Verfahreinrichtung bewegt werden kann.

Eine derart ausgebildete Vorrichtung weist den Vorteil auf, dass die Mastwannen der Vorrichtung dichter beisammen angeordnet werden können und gleichzeitig leichter mit Substrat und/oder Insektenlarven befüllt werden können. Ferner weist eine derart ausgebildete Vorrichtung den Vorteil auf, dass die Insektenlarven vereinfacht automatisiert geerntet werden können. Während des Mästens befinden sich die Mastwannen in den Kragarmregalen und können dort dicht beisammen, d.h. mit einem geringen vertikalen und/oder horizontalen Abstand zueinander angeordnet sein. Zur Befüllung mit Substrat und/oder mit Insektenlarven und/oder zur Ernte der Insektenlarven kann eine Mastwanne mittels der Verfahreinrichtung von der ersten Mastwannenposition, beispielsweise eine Mastwannenposition in dem Kragarmregal, in eine zweite Mastwannenposition, beispielsweise eine Mastwannenposition außerhalb des Kragarmregals, translatorisch bewegt werden. In der zweiten Mastwannenposition kann die Befüllung der Mastwanne mit Substrat und/oder Insektenlarven und/oder die Ernte der Insektenlarven vereinfacht erfolgen.

Das zumindest eine Antriebsmittel kann mit der Verfahreinrichtung verbunden sein.

Das zumindest eine Antriebsmittel kann mit dem ersten Verfahrschlitten und/oder mit dem zweiten Verfahrschlitten verbunden sein und/oder in dem ersten Verfahrschlitten und/oder in dem zweiten Verfahrschlitten angeordnet sein. Wenn die Mastwanne in der ersten Wannenaufnahmeeinrichtung des ersten Verfahrschlittens und/oder in der zweiten Wannenaufnahmeeinrichtung des zweiten Verfahrschlittens aufgenommen ist, steht das Antriebsmittel mit der Mastwanne in einer Wirkverbindung, sodass die Mastwanne von einer ersten Position in eine zweite Position gedreht werden kann, sodass ein Inhalt der Mastwanne in der zweiten Position aus der Mastwanne herausfällt.

Eine derart ausgebildete Vorrichtung weist den Vorteil auf, dass eine Mastwanne sowohl translatorisch als auch rotatorisch bewegt werden kann. Insbesondere kann eine Mastwanne einer derart ausgebildeten Vorrichtung zunächst translatorisch bewegt werden und nach der translatorischen Bewegung rotatorisch bewegt werden. Beispielsweise kann eine Mastwanne zum Ernten der Insektenlarven zunächst aus einer ersten Wannenposition translatorisch in eine zweite Wannenposition bewegt werden. Anschließend kann die Mastwanne von einer ersten Position in eine zweite Position gedreht werden, sodass die Insektenlarven der Mastwanne in der zweiten Position aus der Mastwanne herausfallen.

Alternativ oder zusätzlich kann das zumindest eine Antriebsmittel an einer separaten Dreheinrichtung angeordnet sein und/oder mit dieser verbunden sein. Die Dreheinrichtung ist dazu ausgebildet, die zumindest eine Mastwanne von der ersten Position in die zweite Position zu drehen. Die Verfahreinrichtung kann die zumindest eine Mastwanne in der zweiten Mastwannenposition an die Dreheinrichtung übergeben.

Zumindest zwei oder alle Mastwannen können in einer Wirkverbindung zueinanderstehen, sodass eine Drehung von der ersten Position in die zweite Position erfolgen kann. Dadurch können die Mastwannen in einer Vorrichtung mit einem verminderten technischen Aufwand gedreht werden.

Die Wirkverbindung kann als kinematische Wirkverbindung ausgebildet sein. Die kinematische Wirkverbindung kann als eine Rotationswirkverbindung ausgebildet sein.

Die Wirkverbindung kann über zumindest ein Kopplungsmittel erreicht werden. Das Kopplungsmittel kann als Riemen, Keilriemen, Profilriemen, Kette oder ein sonstiges Mittel zur kinematischen Wirkverbindung erreicht werden. Dadurch können die Mastwannen in einer Vorrichtung mit einem nochmals verminderten technischen Aufwand gedreht werden.

Zumindest zwei oder alle Mastwannen können in zumindest einer horizontalen und/oder in zumindest einer vertikalen Reihe angeordnet sein. Dadurch kann vereinfacht die Wirkverbindung zwischen den Mastwannen erreicht werden.

Eine Reihe im Rahmen dieser Erfindung besteht aus zumindest zwei Mastwannen die nebeneinander und/oder übereinander angeordnet sein können.

Zwei Mastwannen in einer horizontalen und/oder vertikalen Reihe können unmittelbar benachbart zueinander angeordnet sein. Unmittelbar benachbart sind zwei Mastwannen, wenn zwischen den beiden Mastwannen keine weitere Mastwanne mehr angeordnet ist.

Die Vorrichtung kann zwei oder drei oder vier oder fünf oder sechs oder sieben oder mehr horizontale Reihen aufweisen die vertikal zueinander angeordnet sind, wobei in jeder horizontalen Reihe zwei oder drei oder vier oder mehr Mastwannen angeordnet sein können. Mit anderen Worten können die Mastwannen in einer Matrix angeordnet sein, wobei die Größe der Matrix durch die Anzahl der in einer horizontalen Reihe und einer vertikalen Reihe angeordneten Mastwannen bestimmt wird. Dadurch kann nochmals vereinfacht die Wirkverbindung zwischen den Mastwannen erreicht werden.

Gemäß einer bevorzugten Ausführungsform, weist die Vorrichtung zwei horizontal zueinander beabstandet angeordnete Matrixanordnungen von Mastwannen auf. Jede Matrixanordnung von Mastwannen weist zwei oder drei oder vier oder fünf oder sechs oder sieben oder mehr horizontale Reihen auf, die vertikal zueinander angeordnet sind, wobei die horizontalen Reihen jeweils zumindest eine Mastwanne, vorzugsweise zwei oder drei oder mehr unmittelbar zueinander benachbarte Mastwannen aufweisen. Zwischen den zwei horizontal zueinander angeordneten Matrixanordnungen von Mastwannen ist ein Freiraum ausgebildet.

Eine Matrixanordnung von Mastwannen kann ein Kragarmregal aufweisen, in dem die Mastwannen in horizontalen Reihen vertikal zueinander beabstandet angeordnet sind. Mit anderen Worten können zwei oder drei oder mehr Mastwannen in einer horizontalen Reihe auf zumindest zwei Kragarmen des Kragarmregals angeordnet sein. Vertikal dazu beabstandet können wiederum zwei oder drei oder mehr Mastwannen in einer horizontalen Reihe auf zumindest zwei Kragarmen des Kragarmregals angeordnet sein.

Die Verfahreinrichtung kann dazu ausgebildet sein, eine Mastwanne aus einer ersten Mastwannenposition in der Matrixanordnung von Mastwannen in eine zweite Mastwannenposition außerhalb der Matrixanordnung der Mastwannen, vorzugsweise im Bereich des Freiraums zwischen zwei Matrixanordnungen von Mastwannen, translatorisch zu bewegen. In der zweiten Mastwannenposition kann die Verfahreinrichtung dazu ausgebildet sein, die Mastwanne von der ersten Position in die zweite Position zu drehen, sodass ein Inhalt der Mastwanne in der zweiten Position aus der Mastwanne herausfällt.

Eine derart ausgebildete Vorrichtung weist den Vorteil auf, dass die Mastwannen dichter in der Matrixanordnung beisammen angeordnet werden können und gleichzeitig leichter mit Substrat und/oder Insektenlarven befüllt werden können. Ferner können die Insektenlarven vereinfacht automatisiert geerntet werden.

Die Mastwannen in einer horizontalen und/oder vertikalen Reihe können jeweils mit einer unmittelbar benachbarten in der gleichen horizontalen und/oder vertikalen Reihe angeordneten Mastwanne in einer kinematischen Wirkverbindung stehen.

Die erste Mastwanne in einer horizontalen und/oder vertikalen Reihe kann mit der unmittelbar benachbarten Mastwanne in der gleichen horizontalen und/oder vertikalen Reihe in einer kinematischen Wirkverbindung stehen.

Die erste und die letzte in einer horizontalen und/oder vertikalen Reihe angeordnete Mastwanne können ein Kopplungselement am freien Abschnitt des Aufnahmedoms aufweisen. Die restlichen in der gleichen horizontalen und/oder vertikalen Reihe angeordneten Mastwannen können zwei Kopplungselemente am freien Abschnitt des Aufnahmedoms aufweisen. Die erste und die letzte Mastwanne einer horizontalen und/oder vertikalen Reihe weist lediglich eine direkt benachbarte Mastwanne zu einer Seite und/oder nach oben oder nach unten auf.

Nach einer besonders bevorzugten Ausführungsform kann die Vorrichtung sieben horizontale Reihen aufweisen, die untereinander angeordnet sind, wobei jede horizontale Reihe jeweils vier Mastwannen aufweist. Damit ergibt sich eine Anordnung von vier Mastwannen in einer horizontalen Reihe und sieben Mastwannen in einer vertikalen Reihe. Dadurch können die Mastwannen in einer horizontalen Reihe und/oder die Mastwannen in einer vertikalen Reihe besonders einfach miteinander in eine Wirkverbindung gebracht werden.

Nach einer Ausführungsform können vier Mastwannen in einer horizontalen und/oder vertikalen Reihe in einer kinematischen Wirkverbindung zueinanderstehen. Eine als Rotationswirkverbindung ausgebildete kinematische Wirkverbindung kann derart ausgebildet sein, dass bei einer Drehung einer Mastwanne, die restlichen in der gleichen horizontalen und/oder vertikalen Reihe angeordneten Mastwannen ebenfalls gedreht werden. Dadurch können alle Mastwannen in einer horizontalen und/oder vertikalen Reihe um den gleichen Winkel und mit der gleichen Winkelgeschwindigkeit gedreht werden. Dadurch können die Insektenlarven gleichmäßig geerntet werden.

Zumindest ein Antriebsmittel kann mit zumindest zwei oder allen in einer horizontalen Reihe angeordneten Mastwannen in einer Wirkverbindung stehen und/oder zumindest ein Antriebsmittel kann mit zumindest zwei oder allen in einer vertikalen Reihe angeordneten Mastwannen in einer Wirkverbindung stehen. Dadurch können mit einer besonders geringen Anzahl Antriebsmittel alle Mastwannen gedreht werden.

Ein Antriebsmittel kann mit einer ersten oder einer letzten Mastwanne einer horizontalen und/oder vertikalen Reihe durch ein Getriebe oder direkt durch einen elektrischen Motor in einer kinematischen Wirkverbindung stehen und diese Mastwanne antreiben. Diese erste oder letzte Mastwanne kann mit einer unmittelbar benachbarten Mastwanne über ein Kopplungsmittel in einer kinematischen Wirkverbindung stehen und die unmittelbar benachbarte Mastwanne durch das Kopplungsmittel antreiben.

Das Kopplungsmittel kann über ein Kopplungselement der ersten oder der letzten Mastwanne und über ein Kopplungselement an der unmittelbar benachbarten Mastwanne geführt sein.

Die Vorrichtung kann ein oder mehrere Führungselemente zur Führung und Spannung von Kopplungsmitteln aufweisen. Ein Führungselement kann an der Rückseite der Vorderwand und/oder der Rückwand jeweils zwischen zwei unmittelbar benachbarten Mastwannen angeordnet sein. Ein Führungselement kann als Kettenrad, Riemenführung oder ein sonstiges zur Führung oder Spannung eines Kopplungsmittels geeignetes Element ausgebildet sein.

Nach einer besonders bevorzugten Ausführungsform kann genau ein Antriebsmittel mit zumindest zwei oder allen in einer horizontalen Reihe angeordneten Mastwannen in einer Wirkverbindung stehen und/oder genau ein Antriebsmittel kann mit zumindest zwei oder allen in einer vertikalen Reihe angeordneten Mastwannen in einer Wirkverbindung stehen. Dadurch können mit einer nochmals geringeren Anzahl Antriebsmittel alle Mastwannen gedreht werden. Dadurch kann die Vorrichtung besonders kostengünstig hergestellt und betrieben werden.

Das genau eine Antriebsmittel kann mit einer ersten oder einer letzten Mastwanne einer horizontalen und/oder vertikalen Reihe durch ein Getriebe oder direkt durch den elektrischen Motor in einer kinematischen Wirkverbindung stehen und diese Mastwanne antreiben. Die angetriebene Mastwanne kann ihrerseits mit ihren unmittelbar benachbarten Mastwannen in einer kinematischen Wirkverbindung stehen und die unmittelbar benachbarten Mastwannen antreiben. Dadurch kann die Vorrichtung mit einer verringerten Anzahl von Antriebsmitteln betrieben werden und dadurch besonders kostengünstig hergestellt und betrieben werden.

Eine Hüllkurve einer Drehung einer Mastwanne kann eine Hüllkurve einer Drehung einer zu dieser Mastwanne vertikal in Reihe angeordneten Mastwanne zumindest teilweise überdecken. Dadurch können besonders viele Mastwannen in einer Vorrichtung angeordnet werden.

Eine Hüllkurve einer Drehung einer Mastwanne von der ersten Position in die zweite Position kann eine Hüllkurve einer Drehung von der ersten Position in die zweite Position einer zu dieser Mastwanne vertikal in Reihe angeordneten Mastwanne zumindest teilweise überdecken. Mit anderen Worten kann der vertikale Abstand zwischen zwei in einer vertikalen Reihe unmittelbar benachbarten angeordneten Mastwannen geringer als die Breite der Mastwannen sein.

Eine Hüllkurve einer Drehung einer Mastwanne kann eine Hüllkurve einer Drehung einer zu dieser Mastwanne vertikal in Reihe angeordneten Mastwanne tangential berühren. Mit anderen Worten kann der vertikale Abstand zwischen zwei in einer vertikalen Reihe unmittelbar benachbarten angeordneten Mastwannen der Breite der Mastwannen entsprechen.

Alternativ kann eine Hüllkurve einer Drehung einer Mastwanne einen Abstand zu einer Hüllkurve einer Drehung einer zu dieser Mastwanne vertikal in Reihe angeordneten Mastwanne aufweisen. Mit anderen Worten kann der vertikale Abstand zwischen zwei in einer vertikalen Reihe unmittelbar benachbarten angeordneten Mastwannen größer als die Breite der Mastwannen sein. Dadurch können die vertikal in einer Reihe angeordneten Mastwannen gleichzeitig gedreht werden. Dadurch kann die Ernte der Insektenlarven beschleunigt werden.

Eine Hüllkurve einer Drehung der Mastwanne kann eine Hüllkurve einer Drehung einer zu dieser Mastwanne horizontal in Reihe angeordneten Mastwanne tangential berühren oder einen Abstand zu dieser aufweisen. Mit anderen Worten kann der horizontale **Ab**stand zwischen zwei in einer horizontalen Reihe angeordneten unmittelbar benachbarten Mastwannen größer oder gleich der Breite der Mastwannen sein. Dadurch können die Mastwannen in einer horizontalen Reihe gleichzeitig von der ersten Position in die zweite Position gedreht werden. Somit können die Insektenlarven beschleunigt geerntet werden.

Unter der zumindest einen Mastwanne kann ein Fördersystem angeordnet sein, sodass ein Inhalt der zumindest einen Mastwanne aus der zumindest einen Mastwanne in Richtung des Fördersystems herausfällt, wenn die zumindest eine Mastwanne in die zweite Position gedreht wird und/oder sich in der zweiten Position befindet. Dadurch kann die zumindest eine Mastwanne automatisch entleert werden und die Insektenlarven können automatisch geerntet werden.

Alternativ oder zusätzlich kann ein Fördersystem in einem Freiraum zwischen zwei horizontal voneinander beabstandeten Matrixanordnungen von Mastwannen angeordnet sein, sodass ein Inhalt der zumindest einen Mastwanne aus der zumindest einen Mastwanne in Richtung des Fördersystems herausfällt, wenn die zumindest eine Mastwanne in die zweite Position gedreht wird und/oder sich in der zweiten Position befindet.

Das Fördersystem kann an oder in der Trägerstruktur angeordnet sein. Insbesondere kann das Fördersystem mit der Trägerstruktur verbunden sein. Dadurch kann eine Relativverschiebung zwischen der Trägerstruktur und dem Fördersystem vermieden werden, sodass der Inhalt der zumindest einen Mastwanne mit einer verbesserten Genauigkeit auf das Fördersystem entleert werden kann.

Das Fördersystem kann als jedes beliebige System geeignet zur Förderung von Insektenlarven und Rückständen ausgebildet sein. Das Fördersystem kann als Förderband oder als Rollenband oder als Rüttelband ausgebildet sein. Insbesondere kann das Fördersystem als eine abschnittweise Kombination der zuvor genannten Fördersysteme ausgebildet sein.

Die Vorrichtung kann zumindest ein Zusatzaggregat aufweisen, wobei das Zusatzaggregat ausgewählt ist aus der Gruppe bestehend aus einer Befülleinrichtung für Insektenlarven, einer Zuführeinrichtung für Substrat, einer Lüftungseinrichtung, einer Temperiereinrichtung, einer Feuchtigkeitsregeleinrichtung, einer Separationseinrichtung und einem Transportbehälter.

Die Feuchtigkeitsregeleinrichtung kann zumindest einen Feuchtigkeitsmesssensor aufweisen. Zumindest ein Feuchtigkeitsmesssensor kann an zum Messen der Luftfeuchtigkeit der zugeführten Luft eingerichtet sein. Zumindest ein Feuchtigkeitsmesssensor kann zum Messen der Luftfeuchtigkeit der abgeführten Luft eingerichtet sein.

Die Temperiereinrichtung kann eine Temperaturregeleinrichtung zur Regelung der Temperatur in dem Container aufweisen.

Die Vorrichtung kann einen Container aufweisen, wobei die zumindest eine Mastwanne, das zumindest eine Antriebsmittel und die zumindest eine Trägerstruktur in dem Container angeordnet sein können. Dadurch kann die Vorrichtung auch im Außengelände platziert werden und ist vor Witterungseinflüssen geschützt. Dadurch kann ein erhöhter Autarkiegrad erreicht werden.

Das Förderband kann in dem Container angeordnet sein.

Das zumindest eine Zusatzaggregat kann in dem Container angeordnet sein. Insbesondere kann eine Befülleinrichtung für Insektenlarven und/oder eine Zuführeinrichtung für Substrat und/oder eine Lüftungseinrichtung und/oder eine Temperiereinrichtung und/oder eine Separationseinrichtung und/oder ein Transportbehälter in dem Container angeordnet sein. Dadurch kann der Autarkiegrad der Vorrichtung nochmals erhöht werden, insbesondere über eine erweiterte Wertschöpfungskette erhöht werden.

Die Lüftungseinrichtung kann auf dem Container oder neben dem Container angeordnet sein. Die Lüftungseinrichtung kann baulich mit dem Container lösbar oder nicht lösbar verbunden sein.

Die Lüftungseinrichtung kann eine weitere Temperiereinrichtung zur Temperierung der zugeführten Luft aufweisen. Dadurch kann die Temperatur in dem Container verbessert eingestellt werden.

Der Transportbehälter kann eine Temperiereinrichtung zur Temperierung aufweisen.

Der Container kann jede beliebige Form aufweisen, die geeignet ist, eine Vorrichtung nach dem ersten Aspekt der Erfindung aufzunehmen. Der Container kann als 40 Fuß oder als 20 Fuß ISO-Container ausgebildet sein.

Nach einem zweiten Aspekt der Erfindung löst die Aufgabe ein Verfahren zur Aufzucht und Ernte von Insektenlarven, wobei das Verfahren nachfolgende Verfahrensschritte aufweist:
- Bereitstellen zumindest einer Mastwanne mit Substrat und Insektenlarven in der ersten Position;
- Mästen der Insektenlarven in der zumindest einen Mastwanne; und
- Ernten der Insektenlarven durch Drehen der zumindest einen Mastwanne von der ersten Position in eine zweite Position, sodass der Inhalt der zumindest einen Mastwanne in der zweiten Position aus der zumindest einen Mastwanne herausfällt.

Alle zuvor für die Vorrichtung offenbarten Merkmale werden auch für das Verfahren offenbart.

Das Bereitstellen der Mastwanne mit Substrat und Insektenlarven kann durch Zuführen von Substrat in die zumindest eine Mastwanne in der ersten Position erfolgen.

Das Zuführen von Substrat in die zumindest eine Mastwanne kann unter Verwendung einer Zuführeinrichtung für Substrat erfolgen. Das Zuführen von Substrat kann unter Verwendung der Befüllöffnung erfolgen.

Bei dem Substrat kann es sich um zerkleinerte Lebensmittelreste handeln. Insbesondere können die Partikel des Substrats einen Durchmesser von kleiner oder gleich 5 mm oder kleiner oder gleich 3 mm oder kleiner oder gleich 1 mm aufweisen. Die Größe der Partikel des Substrats kann durch Sieben des Substrats durch ein Sieb mit einer entsprechenden Maschenweite bestimmt werden. Die Maschenweite eines Siebes kann beispielsweise über das Messreihenverfahren wie es in DIN ISO 9044 beschrieben ist bestimmt werden. Je stärker das Substrat zerkleinert ist, desto leichter können die Insektenlarven das Substrat verdauen und desto schneller können die Insektenlarven geerntet werden.

Das Substrat weist vorzugsweise einen Startfeuchtigkeitsgehalt von kleiner oder gleich 90 % auf, vorzugsweise von kleiner oder gleich 80 % und besonders bevorzugt von kleiner gleich 70 **%.** Bei einem zu hohen Feuchtigkeitsgehalt kann sich die Sterblichkeit der Insektenlarven in dem Substrat erhöhen. Bei einem Substrat mit dem angegebenen Startfeuchtigkeitsgehalt kann die Qualität der Ernte der Insektenlarven verbessert werden.

Der Startfeuchtigkeitsgehalt des Substrats im Rahmen der Erfindung ist als der Massenprozentanteil Flüssigkeit an der Gesamtmasse an Substrat zum Zeitpunkt der Zuführung des Substrats zu den Mastwannen definiert.

Das Substrat weist vorzugsweise einen Endfeuchtigkeitsgehalt von kleiner oder gleich 60 % auf, vorzugsweise von kleiner oder gleich 50 % und besonders bevorzugt von kleiner gleich 40 %. Bei einem zu hohen Feuchtigkeitsgehalt kann der Inhalt der Mastwanne nicht mehr separiert werden. Bei einem Substrat mit dem angegebenen Endfeuchtigkeitsgehalt kann die Qualität der Ernte der Insektenlarven verbessert werden.

Der Endfeuchtigkeitsgehalt des Substrats im Rahmen der Erfindung ist als der Massenprozentanteil Flüssigkeit an der Gesamtmasse an Substrat zum Zeitpunkt der Ernte der Insektenlarven definiert.

Das Bereitstellen der Mastwanne mit Substrat und Insektenlarven kann durch Befüllen der zumindest einen Mastwanne mit Insektenlarven in der ersten Position erfolgen.

Das Befüllen der zumindest einen Mastwanne kann unter Verwendung einer Befülleinrichtung für Insektenlarven erfolgen. Alternativ oder zusätzlich kann das Befüllen der zumindest einen Mastwanne manuell erfolgen.

Die Insektenlarven können in allen Larvenstadien in die zumindest eine Mastwanne befüllt werden. Abhängig von der Art der verwendeten Insektenlarve, kann die Befüllung zu definierten Larvenstadien vorteilhaft sein. Für die Insektenlarven der Schwarzen Soldatenfliege hat sich die Befüllung der zumindest einen Mastwanne im zweiten Larvenstadium als vorteilhaft erwiesen. Typischerweise erreicht eine Larve der Schwarzen Soldatenfliege das zweite Larvenstadium nach drei bis fünf Tagen nachdem die Larve geschlüpft ist. Eine Larve der Schwarzen Soldatenfliege wiegt typischerweise drei bis fünf Milligramm, wenn sie das zweite Insektenstadium erreicht. Der Fachmann kann abhängig von den verwendeten Insektenlarven und dem verwendeten Substrat ein geeignetes Larvenstadium für die Befüllung der Mastwannen mit Insektenlarven bestimmen. Die Bestimmung des geeigneten Larvenstadiums kann in Abhängigkeit der Dauer nachdem die Larven geschlüpft sind bestimmt werden. Alternativ oder zusätzlich kann das geeignete Larvenstadium in Abhängigkeit des Gewichts der Larven bestimmt werden.

Das Mästen der Insektenlarven kann über einen definierten Zeitraum, der sogenannten Mastzeit, erfolgen. Die Mastzeit ist abhängig von der verwendeten Art der Insektenlarven, dem verwendeten Substrat, den vorliegenden Umgebungsbedingungen am Aufstellort der Vorrichtung und der gewünschten Nährstoffzusammensetzung der Insektenlarven zum Erntezeitpunkt. Für die Soldatenfliege hat sich eine Mastzeit von 7 bis 21 Tagen als vorteilhaft hinsichtlich Zeit- und Energieeinsatz bezogen auf den Ernteertrag erwiesen. Der Fachmann kann in Abhängigkeit der oben genannten Einflussparameter eine geeignete Mastzeit bestimmen.

Das Mästen der Insektenlarven kann bei einem definierten Zuführen und Abführen von Luft erfolgen. Dadurch kann der Feuchtigkeitsgehalt des Substrats definiert eingestellt werden. Dadurch kann erreicht werden, dass das Substrat von einem gegebenen Startfeuchtigkeitsgehalt auf einen benötigten Endfeuchtigkeitsgehalt gebracht werden kann.

Insbesondere kann das definierte Zuführen und Abführen von Luft derart erfolgen, dass die Substratfeuchtigkeit während der Mastzeit von einem Startfeuchtigkeitsgehalt von größer oder gleich 80 % auf einen Endfeuchtigkeitsgehalt von kleiner oder gleich 50 % reduziert werden kann.

Das Zuführen und Abführen von Luft kann unter Verwendung einer Lüftungseinrichtung erfolgen.

Das Zuführen und Abführen von Luft kann die in einem Container befindliche Luft mit einer Rate von größer oder gleich 26 1/h austauschen, vorzugsweise von größer oder gleich 50 1/h, besonders bevorzugt von größer oder gleich 100 1/h und abermals bevorzugt von größer oder gleich 133 1/h. Durch eine derart ausgebildete Luftwechselrate kann der Tod der Insektenlarven verhindert werden. Denn kurz vor der Ernte der Larven produzieren die Insektenlarven viel Wärme. Wird diese Wärme nicht ausreichend schnell abgeführt können die Insektenlarven sterben und die Ernte verschlechtern. Weiterhin kann durch eine derart ausgebildete Luftwechselrate ein sog. "Crawl-Out" der Insektenlarven verhindert werden. Wenn die Luftfeuchtigkeit in dem Container zu hoch wird, kann Wasser an der Mastwanne kondensieren. Die Insektenlarven können dann aufgrund der Oberflächenspannung des kondensierten Wasserst aus den Mastwannen herausklettern.

Das Zuführen und Abführen von Luft kann eine Luftwechselrate in einem Container von größer oder gleich 2000 m³/h erzeugen, vorzugsweise von größer oder gleich 5000 m³/h und besonders bevorzugt von größer oder gleich 10000 m³/h.

Das Zuführen und Abführen von Luft kann einen Luftstrom erzeugen, der durch die Belüftungsöffnungen der Vorderwand und/oder der Rückwand, über die zumindest eine Mastwanne zwischen der Vorderwand und der Rückwand hinweg, durch die Belüftungsöffnungen der Rückwand und/oder der Vorderwand strömt. Dadurch kann der Luftstrom Feuchtigkeit aus dem Substrat beim Überströmen der Mastwannen aufnehmen. Damit kann der Feuchtigkeitsgehalt des Substrats verbessert kontrolliert eingestellt werden.

Das Verfahren kann nach dem Mästen der Insektenlarven in der zumindest einen Mastwanne und vor dem Ernten der Insektenlarven durch Drehen der zumindest einen Mastwanne von der ersten Position in die zweite Position, sodass der Inhalt der zumindest einen Mastwanne in der zweiten Position aus der zumindest einen Mastwanne herausfällt, den folgenden Verfahrensschritt aufweisen:
- Verfahren der zumindest einen Mastwanne von einer ersten Mastwannenposition in eine zweite Mastwannenposition.

Das Verfahren der Mastwanne kann durch eine translatorische Bewegung der Mastwanne erfolgen. Das Verfahren der Mastwanne kann unter Verwendung einer Verfahreinrichtung erfolgen, insbesondere unter Verwendung einer Verfahreinrichtung gemäß dem ersten Aspekt der Erfindung.

Ein derart ausgebildetes Verfahren weist den Vorteil auf, dass die Mastwannen vereinfacht mit Substrat und/oder Instektenlarven befüllt werden können und/oder dass die Insektenlarven vereinfacht geerntet werden können.

Das Verfahren kann den folgenden Verfahrensschritt aufweisen:
- Regeln der Temperatur in dem Container zumindest während dem Mästen der Insektenlarven.

Das Regeln der Temperatur in dem Container kann unter Verwendung einer Temperiereinrichtung erfolgen.

Das Verfahren kann den folgenden Verfahrensschritt aufweisen:
- Regeln der Feuchtigkeit in dem Container zumindest während dem Mästen der Insektenlarven.

Das Regeln der Feuchtigkeit in dem Container kann unter Verwendung einer Feuchtigkeitsregeleinrichtung erfolgen.

Die Feuchtigkeit kann die Luftfeuchtigkeit in dem Container und/oder die Substratfeuchtigkeit des Substrats umfassen.

Das Verfahren kann nach dem Mästen der Insektenlarven und vor dem Ernten der Insektenlarven folgende Verfahrensschritte aufweisen:
- Erneutes Zuführen von Substrat in die zumindest eine Mastwanne in einer ersten Position;
- Weiteres Mästen der Insektenlarven in der zumindest einen Mastwanne.

Dadurch kann eine verbesserte Ausnutzung des Substrats durch die Insektenlarven erreicht werden. Es kann passieren, dass das Substrat anfängt zu faulen, wenn die Insektenlarven das Substrat nicht schnell genug umsetzen und gleichzeitig der Feuchtigkeitsgehalt des Substrats durch die Luftströmung nicht schnell genug reduziert werden kann. Durch ein erneutes Zuführen einer geringeren Menge Substrate und ein weiteres Mästen kann dies vermieden werden.

Das Verfahren kann eine beliebige Anzahl von zusätzlichen Verfahrensschritten des erneuten Zuführens und weiteren Mästens aufweisen. Dadurch kann die Ausnutzung des Substrats definiert eingestellt und verbessert werden. Außerdem kann vermieden werden, dass das Substrat anfängt zu faulen. Dadurch wird eine effizientere Ausnutzung des Substrats erreicht.

Das Ernten der Insektenlarven kann vor dem Puppenstadium oder im Puppenstadium oder zu einem beliebigen anderen Larvenstadium erfolgen. In Abhängigkeit der verwendeten Art der Insektenlarven, dem verwendeten Substrat und der gewünschten Nährstoffzusammensetzung der Insektenlarven kann der Fachmann einen geeigneten Erntezeitpunkt festlegen. Für die Soldatenfliege hat sich das sechste Larvenstadium als vorteilhaft hinsichtlich Nährstoffzusammensetzung und benötigter Mastzeit erwiesen.

Das Drehen der zumindest einen Mastwanne kann mit einer konstanten Winkelgeschwindigkeit erfolgen. Dadurch können die Insektenlarven besonders schonend geerntet werden.

Das Verfahren kann nach dem Ernten der Insektenlarven den folgenden Verfahrensschritt aufweisen:
- Fördern des aus der zumindest einen Mastwanne herausgefallenen Inhalts zu einer Separationseinrichtung.

Dadurch kann das Ernten der Insektenlarven vereinfacht automatisiert durchgeführt werden.

Das Fördern des aus der zumindest einen Mastwanne herausgefallenen Inhalts kann unter Verwendung eines Fördersystems erfolgen.

Das Verfahren kann nach dem Ernten der Insektenlarven den folgenden Verfahrensschritt aufweisen:
- Separieren des Inhalts der zumindest einen Mastwanne in einen Teil Insektenlarven und in einen anderen Teil Rückstände.

Dadurch wird ein erhöhter Reinheitsgrad der Insektenlarven erreicht. Gleichzeitig wird dadurch eine Reduktion der Transportkosten zur Weiterverarbeitung der Insektenlarven erreicht.

Das Separieren kann unter Verwendung einer Separationseinrichtung erfolgen. Die Separationseinrichtung kann als Sieb, als Rüttelsieb oder als sonstige Mittel geeignet zum Trennen von unterschiedlich großen Partikeln ausgebildet sein.

Das Verfahren kann nach dem Separieren den folgenden Verfahrensschritt aufweisen:
- Kühlen der Insektenlarven für den Transport und/oder die Lagerung.

Das Kühlen der Insektenlarven kann auch ein Tiefkühlen der Insektenlarven sein. Dadurch können die Insektenlarven verbessert transportiert werden. Außerdem können die Insektenlarven dadurch verbessert lagerfähig gemacht werden.

Das Verfahren kann nach dem Separieren oder nach dem Kühlen den folgenden Verfahrensschritt aufweisen:
- Einfüllen der Insektenlarven in einen Transportbehälter.

Die Insektenlarven können vor und/oder während und/oder nach dem Einfüllen in den Transportbehälter temperiert werden, insbesondere gekühlt werden, vorzugsweise tiefgekühlt werden. Dadurch werden die Insektenlarven hart genug für eine Lagerung in einem Transportbehälter und können über einen verlängerten Zeitraum gelagert werden.

Das Verfahren erfolgt unter Verwendung einer Vorrichtung gemäß einem der Ansprüche 1 bis 13.

Weitere Vorteile, Einzelheiten und Merkmale der Erfindung ergeben sich nachfolgend aus den erläuterten Ausführungsbeispielen.

Dabei zeigen im Einzelnen:
- Figur 1:: eine schematische Darstellung einer ersten Ausführungsform einer Vorrichtung zur Aufzucht und Ernte von Insekten in einer perspektivischen Ansicht
- Figur 2:: eine schematische Darstellung der ersten Ausführungsform einer Vorrichtung zur Aufzucht und Ernte von Insekten in einer perspektivischen Ansicht der Vorderseite der Rückwand
- Figur 3:: eine schematische Darstellung der ersten Ausführungsform einer Vorrichtung zur Aufzucht und Ernte von Insekten in einer perspektivischen Ansicht der Vorderseite der Vorderwand
- Figur 4:: eine schematische Darstellung der ersten Ausführungsform einer Vorrichtung zur Aufzucht und Ernte von Insekten in einer perspektivischen Ansicht der Rückseite der Vorderwand
- Figur 5:: eine schematische Darstellung einer zweiten Ausführungsform einer Vorrichtung zur Aufzucht und Ernte von Insekten in einer perspektivischen Ansicht

In der nun folgenden Beschreibung bezeichnen gleiche Bezugszeichen gleiche Bauteile bzw. gleiche Merkmale, so dass eine in Bezug auf eine Figur durchgeführte Beschreibung bezüglich eines Bauteils auch für die anderen Figuren gilt

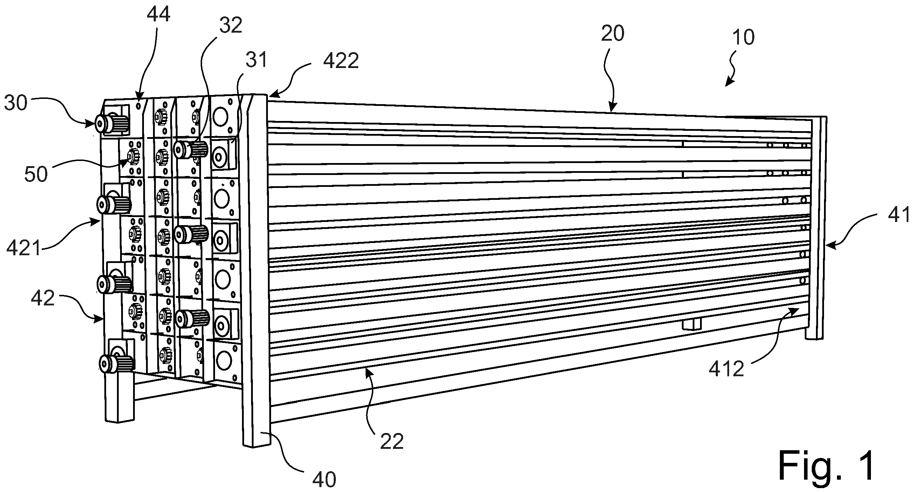

Eine erste Ausführungsform einer Vorrichtung 10 zur Aufzucht und Ernte von Insektenlarven gemäß Figur 1 weist zumindest eine Mastwanne 20, zumindest ein Antriebsmittel 30 und zumindest eine Trägerstruktur 40 auf.

Die dargestellte Vorrichtung 10 zeigt 28 Mastwannen 20 in der ersten Position, wobei die Mastwannen 20 in sieben horizontalen Reihen mit jeweils vier in einer horizontalen Reihe angeordneten Mastwanen 20 angeordnet sind. Die sieben horizontalen Reihen sind vertikal zueinander angeordnet, sodass sich eine Matrixstruktur aus vier Mastwannen 20 in einer horizontalen Reihe und sieben Mastwannen 20 in einer vertikalen Reihe ergibt.

Die Mastwannen 20 sind in der Trägerstruktur 40 zwischen der Vorderwand 41 und der Rückwand 42 der Trägerstruktur 40 aufgenommen. Die Rückseite 412 der Vorderwand 41 liegt dabei der Rückseite 422 der Rückwand 42 gegenüber. Die Mastwannen 20 weisen Aufnahmedome 23 auf, die an der die Länge der Mastwannen 20 begrenzenden Seitenwand angeordnet sind und orthogonal von der Seitenwand von dem Mastwannenvolumen 21 weg ragen. Die Aufnahmedome 23 sind in Aufnahmeeinrichtungen 50 in der Vorderwand 41 und der Rückwand 42 der Trägerstruktur 40 aufgenommen. Die Aufnahmedome 23 erstrecken sich von der Rückseite 422 der Rückwand 42 durch die Rückwand 42 hindurch sodass der freie Abschnitt 24 des Aufnahmedoms 23 orthogonal von der Vorderseite 421 der Rückwand 42 weg ragt.

Die Vorderwand 41 und die Rückwand 42 weisen Belüftungsöffnungen 44 auf. Die Belüftungsöffnungen 44 sind oberhalb der Seitenwände der Mastwannen 20 angeordnet.

Die Vorrichtung 10 weist sieben Antriebsmittel 30 auf, wobei jeweils genau ein Antriebsmittel 30 mit jeweils vier in einer horizontalen Reihe angeordneten Mastwannen 20 in einer kinematischen Wirkverbindung steht. Das Antriebsmittel 30 ist dazu eingerichtet, die mit dem Antriebsmittel 30 in einer kinematischen Wirkverbindung stehenden Mastwannen 20 um die Längsachse der Mastwannen 20 von der ersten Position in die zweite Position zu drehen. Die Antriebsmittel 30 weisen elektrische Motoren 32 und Schneckengetriebe 31 auf. Die Antriebsmittel 30 sind an der Rückwand 42 angeordnet, wobei der elektrische Motor 32 eine Mastwanne 20 über ein Schneckengetriebe 31 antreibt. Das Schneckengetriebe ist mit dem freien Abschnitt 24 des Aufnahmedoms 23 der Mastwanne 20 verbunden.

Die Mastwannen 20 weisen eine Verstärkungsstruktur 22 auf, die am Boden der Mastwannen 20 angeordnet ist. Die Verstärkungsstruktur 22 verläuft in Längsrichtung der Mastwannen 20 und weist ein Doppel-T-Trägerprofil auf.

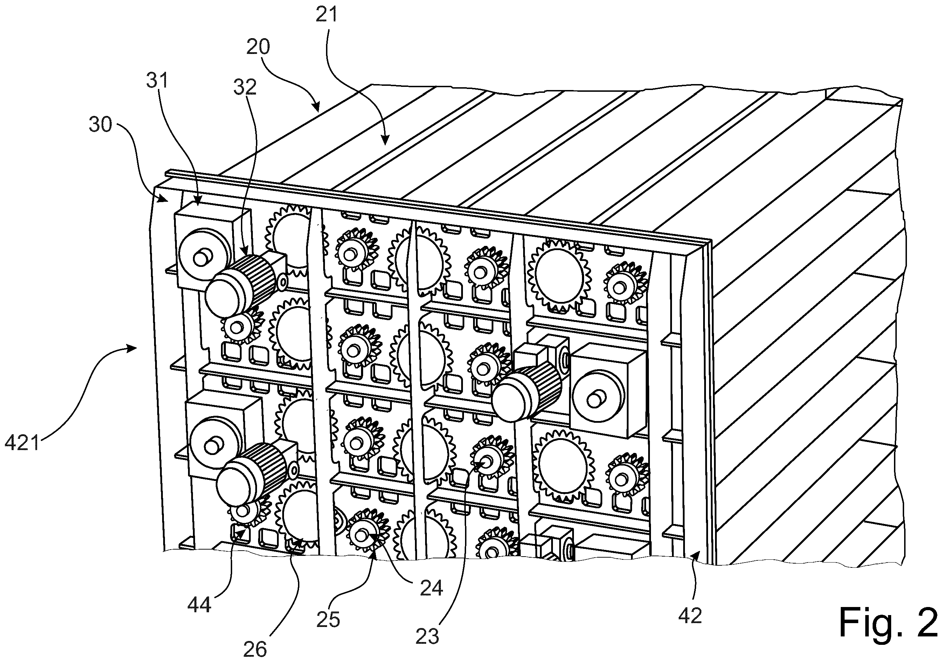

Gemäß Figur 2 weist die erste Ausführungsform der Vorrichtung 10 genau ein Antriebsmittel 30 in einer horizontalen Reihe von Mastwannen 20 auf. Das Antriebsmittel 30 ist an der ersten oder der letzten Mastwanne 20 einer horizontalen Reihe angeordnet. Das Antriebsmittel 30 weist ein Schneckengetriebe 31 und einen elektrischen Motor 32 auf. Das Schneckengetriebe 31 ist mit dem freien Abschnitt 24 der ersten oder letzten Mastwanne 20 verbunden, sodass der elektrische Motor 32 die Mastwanne 20 über das Schneckengetriebe 31 von der ersten Position in die zweite Position drehen kann. Die erste und die letzte Mastwanne 20 einer horizontalen Reihe weist ein Kopplungselement 25 auf, welches an dem freien Abschnitt 24 des Aufnahmedoms 23 angeordnet ist. Die mittleren beiden Mastwannen 20 weisen jeweils zwei Kopplungselemente 25 auf, die koaxial zueinander auf einem freien Abschnitt 24 eines Aufnahmedoms 23 einer Mastwanne 20 beabstandet zueinander angeordnet sind. Die Kopplungselemente 25 sind als Kettenräder ausgebildet. Zwischen zwei Mastwannen 20 der horizontalen Reihe ist ein Führungselement 26 zur Führung und Spannung des Kopplungsmittels 51 angeordnet. Das Führungselement 26 ist als Spannkettenrad ausgebildet. Jeweils zwei unmittelbar benachbarte Mastwannen 20 stehen durch ein Kopplungsmittel 51 in einer kinematischen Wirkverbindung zueinander. Das Kopplungsmittel 51 verbindet die beiden Kopplungselemente 25 der unmittelbar benachbarten Mastwannen 20 miteinander, sodass die Drehung einer Mastwanne 20 von der ersten Position in die zweite Position unmittelbar die Drehung der unmittelbar benachbarten Mastwanne 20 bewirkt. Das Kopplungsmittel 25 ist als Kette ausgebildet.

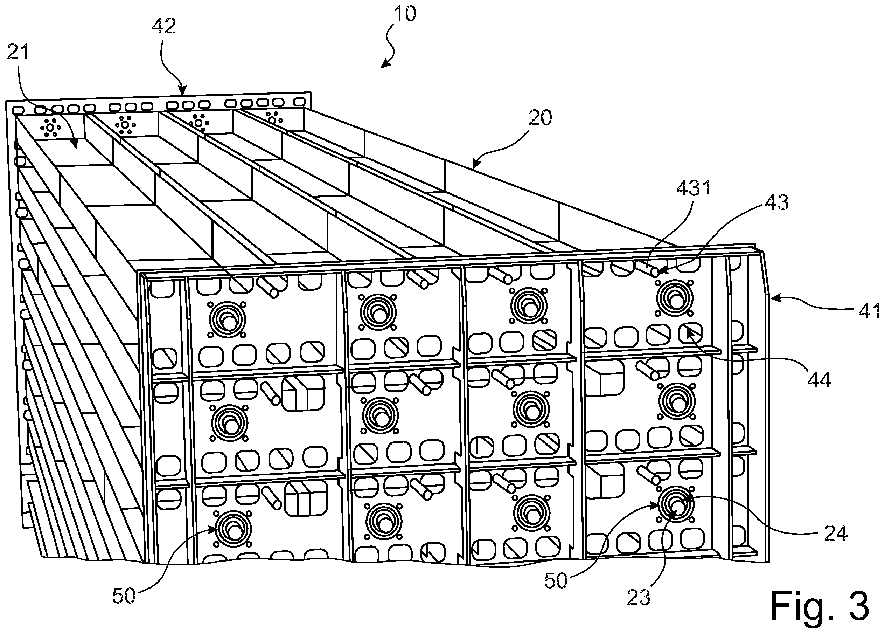

Gemäß Figur 3 weist die erste Ausführungsform einer Vorrichtung 10 zur Aufzucht und Ernte von Insektenlarven Mastwannen 20 in der ersten Position mit einem Boden und vier Seitenwänden auf, sodass ein Mastwannenvolumen 21 von Boden und Seitenwänden teilweise begrenzt wird. Die Mastwannen 20 sind nach oben hin offen, sodass beim Drehen der Mastwannen 20 von der ersten Position in die zweite Position ein Inhalt der Mastwannen 20 aus den Mastwannen 20 herausfällt. Die Mastwannen 20 weisen eine quaderförmige Form auf.

Die Mastwannen 20 sind durch die Aufnahmeeinrichtungen 50 in der Vorderwand 41 der Trägerstruktur 40 aufgenommen. Die Aufnahmedome 23 der Mastwannen 20 ragen von der Rückseite 412 der Vorderwand 41 durch die Vorderwand 41 hindurch sodass die freien Abschnitte 24 der Aufnahmedome 23 von der Vorderseite 411 der Vorderwand 41 orthogonal weg ragen.

Der Querschnitt einer Belüftungsöffnung 44 der Vorderwand 41 überdeckt den Querschnitt genau einer gegenüberliegenden Belüftungsöffnung 44 der Rückwand 42, sodass ein paar korrespondierender Belüftungsöffnungen 44 gebildet wird. Durch ein solches Paar korrespondierender Belüftungsöffnungen 44 kann ein Luftstrom durch die Belüftungsöffnung 44 der Rückwand 42 über eine Mastwanne 20 zwischen der Vorderwand 41 und der Rückwand 42 durch die Belüftungsöffnung 44 der Vorderwand 41 geführt werden. Die Vorrichtung weist eine Vielzahl von Belüftungsöffnungen 44 in der Vorderwand 41 und der Rückwand 42 der Trägerstruktur 40 auf, wobei jeweils zwei sich gegenüberliegende Belüftungsöffnungen 44 ein Paar korrespondierender Belüftungsöffnungen 44 bildet. Die Belüftungsöffnungen 44 sind oberhalb der Seitenwände der Mastwannen 20 in der ersten Position angeordnet, sodass ein Luftstrom über die Mastwannen 20 geführt werden kann.

Je Mastwanne 20 ist eine Befüllöffnung 43 an der Vorderwand 41 oberhalb der Seitenwände der Mastwanne 20 in der ersten Position angeordnet. Die Befüllöffnungen 43 sind jeweils parallel zur Längsachse der Mastwanne 20 angeordnet, sodass die Befüllöffnung 43 mittig bezogen auf die Breite der Mastwanne oberhalb der Mastwanne angeordnet ist. Die Befüllöffnung 43 ist als Rohr ausgebildet und weist ein Anschlusselement 431 auf, an welches eine Zuführeinrichtung und/oder an eine Befülleinrichtung angeschlossen werden kann. Das Anschlusselement 431 ragt orthogonal von der Vorderseite 411 der Vorderwand 41 weg.

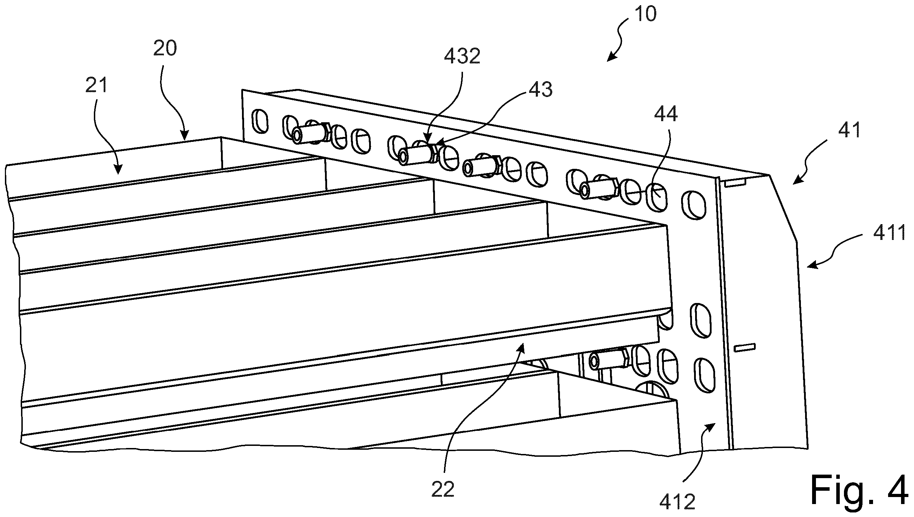

Gemäß Figur 4 weist die Befüllöffnung 43 der ersten Ausführungsform einer Vorrichtung 10 zur Aufzucht und Ernte von Insektenlarven, ein Abgabeelement 432 auf, welches an der Rückseite 412 der Vorderwand 41 angeordnet ist und von der Rückseite 412 der Vorderwand 41 orthogonal weg ragt, sodass das Abgabeelement 432 mittig bezogen auf die Breite der Mastwanne über der Mastwanne 20 in der ersten Position ragt. Jeder Mastwanne 20 ist genau eine Befüllöffnung 43 und damit genau ein Abgabeelement 432 zugeordnet, sodass jeder Mastwanne 20 durch die jeweils zugeordnete Befüllöffnung 43 Substrat zugeführt werden kann und/oder jede Mastwanne 20 durch die jeweils zugeordnete Befüllöffnung 43 mit Insektenlarven befüllt werden kann. Das Abgabeelement 432 ist als Rohr ausgebildet.

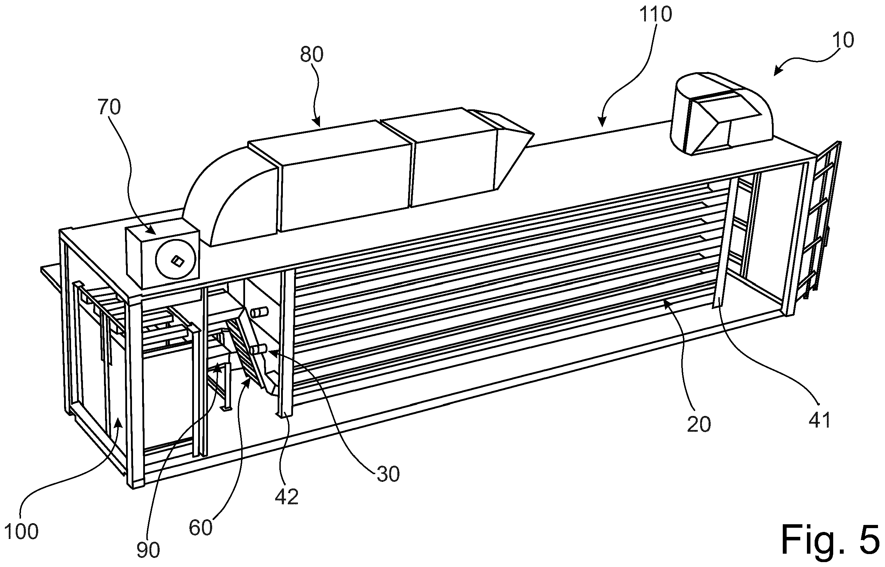

Eine zweite Ausführungsform einer Vorrichtung 10 zur Aufzucht und Ernte von Insektenlarven gemäß Figur 5 weist einen Container 110 auf, wobei die Trägerstruktur 40, die Antriebsmittel 30 und die Mastwannen 20 in dem Container 110 angeordnet sind.

Ferner weist die Vorrichtung ein Fördersystem 60 auf. Das Fördersystem 60 ist unterhalb der Mastwannen 20 an der Trägerstruktur 40 angeordnet und ebenfalls in dem Container 110 angeordnet. Das Fördersystem 60 ist als Förderband ausgebildet.

Ferner weist die Vorrichtung 10, eine Temperiereinrichtung 70, eine Lüftungseinrichtung 80, eine Separationseinrichtung 90 und zwei Transportbehälter 100 auf. Die Separationseinrichtung 90 und die zwei Transportbehälter 100 sind ebenfalls in dem Container 110 angeordnet. Die Lüftungseinrichtung 80 und die Temperiereinrichtung 70 sind auf dem Container angeordnet.

Die Lüftungseinrichtung 80 saugt Umgebungsluft an und führt sie dem Container zu, sodass ein Luftstrom durch die Belüftungsöffnungen 44 der Vorderwand 41 über die Mastwannen 20 durch die Belüftungsöffnungen 44 der Rückwand geführt wird. Der aus den Belüftungsöffnungen 44 der Rückwand austretende Luftstrom wird durch die Lüftungseinrichtung 80 wieder aus dem Container 110 abgeführt.

Die zwei Transportbehälter 100 können durch die Temperiereinrichtung 70 derart temperiert werden, dass Insektenlarven die in die Transportbehälter 100 transportiert werden ausreichend für eine Lagerung gekühlt sind insbesondere tiefgekühlt sind.

### Bezugszeichenliste

- 10: Vorrichtung
- 20: Mastwanne
- 21: Mastwannenvolumen
- 22: Verstärkungsstruktur
- 23: Aufnahmedom
- 24: Freier Abschnitt (des Aufnahmedoms)
- 25: Kopplungselement
- 26: Führungselement
- 30: Antriebsmittel
- 31: Schneckengetriebe
- 32: Elektrischer Motor
- 40: Trägerstruktur
- 41: Vorderwand
- 411: Vorderseite der Vorderwand
- 412: Rückseite der Vorderwand
- 42: Rückwand
- 421: Vorderseite der Rückwand
- 422: Rückseite der Rückwand
- 43: Befüllöffnung
- 431: Anschlusselement
- 432: Abgabeelement
- 44: Belüftungsöffnung
- 50: Aufnahmeeinrichtung
- 51: Kopplungsmittel
- 60: Fördersystem
- 70: Temperiereinrichtung
- 80: Lüftungseinrichtung
- 90: Separationseinrichtung
- 100: Transportbehälter
- 110: Container

## Patentansprüche

1. Vorrichtung (10) zur Aufzucht und Ernte von Insektenlarven aufweisend:
- zumindest eine Mastwanne (20);
- zumindest eine Trägerstruktur (40);
- zumindest ein Antriebsmittel (30);
wobei
- die Mastwanne (20) einen Boden und zumindest eine Seitenwand aufweist und der Boden und die Seitenwand ein Mastwannenvolumen (21) begrenzen;
- die Mastwanne (20) eine Drehachse aufweist;
- das Antriebsmittel (30) und die zumindest eine Mastwanne (20) in einer Wirkverbindung zueinanderstehen, sodass die zumindest eine Mastwanne (20) um die Drehachse von einer ersten Position in eine zweite Position gedreht werden kann, sodass ein Inhalt der Mastwanne (20) in der zweiten Position aus der Mastwanne (20) herausfällt;
- wobei die zweite Position der Mastwanne um einen Winkel von größer oder gleich 135 ° zu der ersten Position versetzt ist; und
- die Drehachse der Mastwanne koaxial zur Längsachse der Mastwanne verläuft.

2. Vorrichtung (10) nach einem der vorangegangenen Ansprüche, **dadurch gekennzeichnet, dass** zumindest eine Mastwanne (20) in der Trägerstruktur (40) aufgenommen ist.

3. Vorrichtung (10) nach einem der vorangegangenen Ansprüche, **dadurch gekennzeichnet, dass** die Vorrichtung (10) zumindest eine Aufnahmeeinrichtung (50) aufweist, wobei die zumindest eine Mastwanne (20) über die zumindest eine Aufnahmeeinrichtung (50) in der Trägerstruktur (40) aufgenommen ist.

4. Vorrichtung (10) nach einem der vorangegangenen Ansprüche, **dadurch gekennzeichnet, dass** die Vorrichtung (10) zwei oder mehr Mastwannen (20) aufweist.

5. Vorrichtung (10) nach einem der vorangegangenen Ansprüche, **dadurch gekennzeichnet, dass** die Vorrichtung (10) eine Verfahreinrichtung aufweist, wobei die Verfahreinrichtung dazu ausgebildet ist, die zumindest eine Mastwanne (20) in horizontaler und/oder vertikaler Richtung translatorisch zu bewegen.

6. Vorrichtung (10) nach Anspruch 5, **dadurch gekennzeichnet, dass** das zumindest eine Antriebsmittel (30) mit der Verfahreinrichtung verbunden ist und/oder an der Verfahreinrichtung angeordnet ist.

7. Vorrichtung (10) nach Anspruch 4, **dadurch gekennzeichnet, dass** zumindest zwei oder alle Mastwannen (20) in einer Wirkverbindung zueinanderstehen, sodass eine Drehung von der ersten Position in die zweite Position erfolgen kann, wobei die Wirkverbindung als kinematische Wirkverbindung ausgebildet ist.

8. Vorrichtung (10) nach einem der Ansprüche 4 bis 7, **dadurch gekennzeichnet, dass** zumindest zwei oder alle Mastwannen (20) in zumindest einer horizontalen und/oder in zumindest einer vertikalen Reihe angeordnet sind.

9. Vorrichtung (10) nach Anspruch 8, **dadurch gekennzeichnet, dass**
- zumindest ein Antriebsmittel (30) mit zumindest zwei oder allen in einer horizontalen Reihe angeordneten Mastwannen (20) in einer Wirkverbindung steht;
und/oder
- zumindest ein Antriebsmittel (30) mit zumindest zwei oder allen in einer vertikalen Reihe angeordneten Mastwannen (20) in einer Wirkverbindung steht.

10. Vorrichtung (10) nach einem der Ansprüche 8 oder 9, **dadurch gekennzeichnet, dass** eine Hüllkurve einer Drehung einer Mastwanne (20) eine Hüllkurve einer Drehung einer zu dieser Mastwanne (20) vertikal in Reihe angeordneten Mastwanne (20) zumindest teilweise überdeckt.

11. Vorrichtung (10) nach einem der vorangegangenen Ansprüche, **dadurch gekennzeichnet, dass** ein Fördersystem (60) unter der zumindest einen Mastwanne (20) angeordnet ist, sodass ein Inhalt der zumindest einen Mastwanne (20) aus der zumindest einen Mastwanne (20) in Richtung des Fördersystems (60) herausfällt wenn die zumindest eine Mastwanne (20) in die zweite Position gedreht wird und/oder sich in der zweiten Position befindet.

12. Vorrichtung (10) nach einem der vorangegangenen Ansprüche, aufweisend zumindest ein Zusatzaggregat, ausgewählt aus der Gruppe bestehend aus
- einer Befülleinrichtung für Insektenlarven; und/oder
- einer Zuführeinrichtung für Substrat; und/oder
- einer Lüftungseinrichtung (80) und/oder;
- einer Temperiereinrichtung (70) und/oder;
- einer Feuchtigkeitsregeleinrichtung und/oder;
- einer Separationseinrichtung (90) und/oder;
- einem Transportbehälter (100).

13. Vorrichtung (10) nach einem der vorangegangenen Ansprüche aufweisend einen Container (110), wobei die zumindest eine Mastwanne (20), das zumindest eine Antriebsmittel und die zumindest eine Trägerstruktur (40) in dem Container (110) angeordnet sind.

14. Verfahren zur Aufzucht und Ernte von Insektenlarven unter Verwendung einer Vorrichtung (10) nach einem der Ansprüche 1 bis 13, aufweisend nachfolgende Verfahrensschritte:
- Bereitstellen zumindest einer Mastwanne mit Substrat und Insektenlarven in einer ersten Position;
- Mästen der Insektenlarven in der zumindest einen Mastwanne (20); und
- Ernten der Insektenlarven durch Drehen der zumindest einen Mastwanne (20) von der ersten Position in eine zweite Position, sodass der Inhalt der zumindest einen Mastwanne (20) in der zweiten Position aus der zumindest einen Mastwanne (20) herausfällt, wobei die zweite Position der Mastwanne um einen Winkel von größer oder gleich 135 ° zu der ersten Position versetzt ist.

## Claims

1. Device (10) for rearing and harvesting insect larvae, comprising:
- at least one fattening trough (20);
- at least one support structure (40);
- at least one drive means (30);
wherein
- the fattening trough (20) has a base and at least one side wall and the base and the side wall delimit a fattening trough volume (21);
- the fattening trough (20) has an axis of rotation;
- the drive means (30) and the at least one fattening trough (20) are operatively connected to one another, so that the at least one fattening trough (20) can be rotated about the axis of rotation from a first position to a second position, so that a content of the fattening trough (20) falls out of the fattening trough (20) in the second position;
- wherein the second position of the fattening trough is offset by an angle of greater than or equal to 135° from the first position; and
- the axis of rotation of the fattening trough runs coaxially with the longitudinal axis of the fattening trough.

2. Device (10) according to any of the preceding claims, **characterized in that** at least one fattening trough (20) is received in the support structure (40).

3. Device (10) according to either of the preceding claims,
**characterized in that** the device (10) has at least one receiving apparatus (50), the at least one fattening trough (20) being received in the support structure (40) via the at least one receiving apparatus (50).

4. Device (10) according to any of the preceding claims,
**characterized in that** the device (10) has two or more fattening troughs (20).

5. Device (10) according to any of the preceding claims,
**characterized in that** the device (10) has a displacement apparatus, the displacement apparatus being designed to move the at least one fattening trough (20) translationally in the horizontal and/or vertical direction.

6. Device (10) according to claim 5, **characterized in that** the at least one drive means (30) is connected to the displacement apparatus and/or is arranged on the displacement apparatus.

7. Device (10) according to claim 4, **characterized in that** at least two or all fattening troughs (20) are operatively connected to one another, so that rotation from the first position to the second position can take place, the operative connection being designed as a kinematic operative connection.

8. Device (10) according to any of claims 4 to 7, **characterized in that** at least two or all fattening troughs (20) are arranged in at least one horizontal and/or at least one vertical row.

9. Device (10) according to claim 8, **characterized in that**
- at least one drive means (30) is operatively connected to at least two or all fattening troughs (20) arranged in a horizontal row;
and/or
- at least one drive means (30) is operatively connected to at least two or all fattening troughs (20) arranged in a vertical row.

10. Device (10) according to either of claims 8 or 9, **characterized in that** an envelope curve of a rotation of a fattening trough (20) at least partially covers an envelope curve of a rotation of a fattening trough (20) arranged vertically in series with this fattening trough (20).

11. Device (10) according to any of the preceding claims,
**characterized in that** a conveyor system (60) is arranged under the at least one fattening trough (20), so that a content of the at least one fattening trough (20) falls out of the at least one fattening trough (20) in the direction of the conveyor system (60) when the at least one fattening trough (20) is rotated into the second position and/or is in the second position.

12. Device (10) according to any of the preceding claims, comprising at least one additional unit selected from the group consisting of
- a filling apparatus for insect larvae; and/or
- a feed apparatus for substrate; and/or
- a ventilation apparatus (80) and/or;
- a temperature control apparatus (70) and/or;
- a humidity control apparatus and/or;
- a separation apparatus (90) and/or;
- a transport container (100).

13. Device (10) according to any of the preceding claims comprising a container (110), wherein the at least one fattening trough (20), the at least one drive means and the at least one support structure (40) are arranged in the container (110).

14. Method for rearing and harvesting insect larvae using a device (10) according to any of claims 1 to 13, comprising the following method steps:
- providing at least one fattening trough with substrate and insect larvae in a first position;
- fattening the insect larvae in the at least one fattening trough (20); and
- harvesting the insect larvae by rotating the at least one fattening trough (20) from the first position to a second position, so that the contents of the at least one fattening trough (20) fall out of the at least one fattening trough (20) in the second position, wherein the second position of the fattening trough is offset at an angle of greater than or equal to 135° from the first position.

## Revendications

1. Dispositif (10) pour l'élevage et la récolte de larves d'insectes présentant :
- au moins une auge d'engraissement (20) ;
- au moins une structure de support (40) ;
- au moins un moyen d'entraînement (30) ;
dans lequel
- l'auge d'engraissement (20) présente un fond et au moins une paroi latérale et le fond et la paroi latérale délimitent un volume d'auge d'engraissement (21) ;
- l'auge d'engraissement (20) présente un axe de rotation ;
- le moyen d'entraînement (30) et l'au moins une auge d'engraissement (20) sont en liaison fonctionnelle l'un avec l'autre, de sorte que l'au moins une auge d'engraissement (20) peut tourner autour de l'axe de rotation d'une première position vers une seconde position, de sorte qu'un contenu de l'auge d'engraissement (20) tombe de l'auge d'engraissement (20) dans la seconde position ;
- dans lequel la seconde position de l'auge d'engraissement est décalée d'un angle supérieur ou égal à 135° par rapport à la première position ; et
- l'axe de rotation de l'auge d'engraissement s'étend de manière coaxiale par rapport à l'axe longitudinal de l'auge d'engraissement.

2. Dispositif (10) selon l'une des revendications précédentes, **caractérisé en ce qu'**au moins une auge d'engraissement (20) est reçue dans la structure de support (40).

3. Dispositif (10) selon l'une des revendications précédentes,
**caractérisé en ce que** le dispositif (10) présente au moins un appareil de réception (50), dans lequel l'au moins une auge d'engraissement (20) est reçue dans la structure de support (40) par l'intermédiaire de l'au moins un appareil de réception (50).

4. Dispositif (10) selon l'une des revendications précédentes,
**caractérisé en ce que** le dispositif (10) présente deux auges d'engraissement (20) ou plus.

5. Dispositif (10) selon l'une des revendications précédentes,
**caractérisé en ce que** le dispositif (10) présente un appareil de déplacement, dans lequel l'appareil de déplacement est conçu pour déplacer en translation l'au moins une auge d'engraissement (20) dans des directions horizontale et/ou verticale.

6. Dispositif (10) selon la revendication 5, **caractérisé en ce que** l'au moins un moyen d'entraînement (30) est relié à l'appareil de déplacement et/ou est disposé sur l'appareil de déplacement.

7. Dispositif (10) selon la revendication 4, **caractérisé en ce qu'au** moins deux auges d'engraissement ou toutes les auges d'engraissement (20) sont en liaison fonctionnelle les unes avec les autres, de sorte qu'une rotation de la première position à la seconde position peut être effectuée, dans lequel la liaison fonctionnelle est conçue comme une liaison fonctionnelle cinématique.

8. Dispositif (10) selon l'une des revendications 4 à 7, **caractérisé en ce qu'au** moins deux auges d'engraissement ou toutes les auges d'engraissement (20) sont disposées en au moins une rangée horizontale et/ou en au moins une rangée verticale.

9. Dispositif (10) selon la revendication 8, **caractérisé en ce que**
- au moins un moyen d'entraînement (30) est en liaison fonctionnelle avec au moins deux auges d'engraissement ou avec toutes les auges d'engraissement (20) disposées en une rangée horizontale ;
et/ou
- au moins un moyen d'entraînement (30) est en liaison fonctionnelle avec au moins deux auges d'engraissement ou avec toutes les auges d'engraissement (20) disposées en une rangée verticale.

10. Dispositif (10) selon l'une des revendications 8 ou 9, **caractérisé en ce qu'**une courbe enveloppante d'une rotation d'une auge d'engraissement (20) recouvre au moins partiellement une courbe enveloppante d'une rotation d'une auge d'engraissement (20) disposée en une rangée verticalement par rapport à ladite auge d'engraissement (20).

11. Dispositif (10) selon l'une des revendications précédentes, **caractérisé en ce qu'**un système de transport (60) est disposé sous l'au moins une auge d'engraissement (20), de sorte qu'un contenu de l'au moins une auge d'engraissement (20) tombe de l'au moins une auge d'engraissement (20) en direction du système de transport (60) lorsque l'au moins une auge d'engraissement (20) tourne vers la seconde position et/ou se trouve dans la seconde position.

12. Dispositif (10) selon l'une des revendications précédentes, présentant au moins un groupe auxiliaire choisi dans le groupe constitué
- d'un appareil de remplissage pour les larves d'insectes ; et/ou
- d'un appareil d'alimentation en substrat ; et/ou
- d'un appareil de ventilation (80) et/ou ;
- d'un appareil de régulation de la température (70) et/ou ;
- d'un appareil de régulation de l'humidité et/ou
- d'un appareil de séparation (90) et/ou ;
- d'un récipient de transport (100).

13. Dispositif (10) selon l'une des revendications précédentes présentant un contenant (110), dans lequel l'au moins une auge d'engraissement (20), l'au moins un moyen d'entraînement et l'au moins une structure de support (40) sont disposés dans le contenant (110).

14. Procédé pour l'élevage et la récolte de larves d'insectes à l'aide d'un dispositif (10) selon l'une des revendications 1 à 13, présentant les étapes de procédé suivantes :
- fourniture d'au moins une auge d'engraissement avec un substrat et des larves d'insectes dans une première position ;
- engraissement des larves d'insectes dans l'au moins une auge d'engraissement (20) ; et
- récolte des larves d'insectes en faisant tourner l'au moins une auge d'engraissement (20) de la première position vers une seconde position, de sorte que le contenu de l'au moins une auge d'engraissement (20) tombe de l'au moins une auge d'engraissement (20) dans la seconde position, dans lequel la seconde position de l'auge d'engraissement est décalée d'un angle supérieur ou égal à 135° par rapport à la première position.
